# EUROPEAN PATENT APPLICATION

(11) **EP 0 737 676 A1**
(43) Date of publication of application: **16.10.1996**
(21) Application number: 95904003.1
(22) Date of filing: 27.12.1994
(51) Int. Cl.: C07C 405/00, A61K 31/557

(54) **PROSTAGLANDIN DERIVATIVE, SALT THEREOF, AND USE THEREOF**

(30) Priority: 29.12.1993 JP 353777/93; 05.04.1994 JP 90664/94
(71) Applicant: TAISHO PHARMACEUTICAL CO. LTD, Tokyo 171 (JP)
(72) Inventor: SATO, Fumie 1-219, Kugenumahigashi 3-chome, Kanawaga 251 (JP); AMANO, Takehiro Taisho Pharmaceutical Co., Ltd., Tokyo 171 (JP); KAMEO, Kazuya Taisho Pharmaceutical Co., Ltd., Tokyo 171 (JP); TANAMI, Tohru Taisho Pharmaceutical Co., Ltd., Tokyo 171 (JP); MUTOH, Masaru Taisho Pharmaceutical Co., Ltd., Tokyo 171 (JP); ONO, Naoya Taisho Pharmaceutical Co., Ltd., Tokyo 171 (JP); GOTO, Jun Taisho Pharmaceutical Co., Ltd., Tokyo 171 (JP)
(74) Representative: Weisert, Annekäte, Dipl.-Ing. Dr.-Ing.
(86) International application number: JP9402267
(87) International publication number: WO9518101

(57) **Abstract**

Prostaglandin derivative represented by Formula (I): 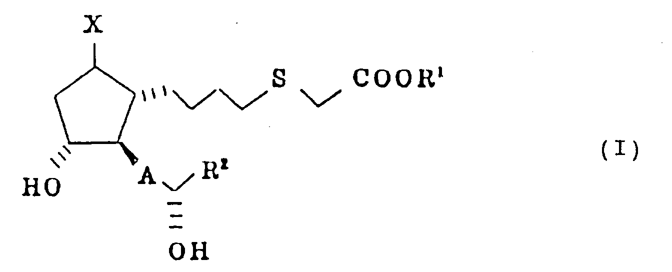 wherein R¹ is a hydrogen atom, an alkyl group having 1 to 10 carbon atoms or a cycloalkyl group having 3 to 10 carbon atoms, and R² is a cycloalkyl group having 3 to 10 carbon atoms, a cycloalkyl group having 3 to 10 carbon atoms substituted by a methyl group, an alkyl group having 1 to 3 carbon atoms substituted by a cycloalkyl group having 3 to 10 carbon atoms, a branched alkyl group having 5 to 10 carbon atoms or a branched alkenyl group having 5 to 10 carbon atoms, X is a halogen atom substituted at the α- or β-configuration, and A is an ethylene group, a vinylene group or an ethynylene group, salts thereof, and a pharmaceutical composition for lowering intraocular pressure containing them as an effective ingredient.

These compounds are useful as lowering agents of intraocular pressure with no or remarkably reduced side-effects such as transient ocular hypertension and the like.

## Description

### TECHNICAL FIELD

The present invention relates to novel prostaglandin (hereinafter referred to as PG) derivatives, salts thereof and use thereof. More particularly, the present invention relates to novel PG derivatives and salts thereof having an excellent property as a lowering agent of intraocular pressure, and use thereof.

### BACKGROUND ART

Since PGs and derivatives thereof exhibit various important pharmaceutical and physiological actions in a trace amount, investigations have been made of the synthesis and biological activity of natural PGs and a large number of PG derivatives with the intention of use as medicines. These PGs and derivatives thereof are known to have a vasodilating action, an inflammating action, an inhibiting action of blood platelet aggregation, a uterine muscle contraction action and an intestinal contraction action as the pharmaceutical and physiological actions. Among these compounds are included those having a lowering action of intraocular pressure and being effective to glaucoma. Many of these compounds, however, have side-effects such as transient ocular hypertension, lacrimation, lid closure or iridal hyperemia, therefore it is a problem to use them as medicines.

An object of the present invention is to provide PG derivatives and salts thereof useful as lowering agents of intraocular pressure. A further object of the present invention is to provide lowering agents of intraocular pressure with no or extremely reduced side-effects such as transient ocular hypertension and the like.

### DISCLOSURE OF THE INVENTION

The present invention is to provide novel PG derivatives represented by Formula (I): 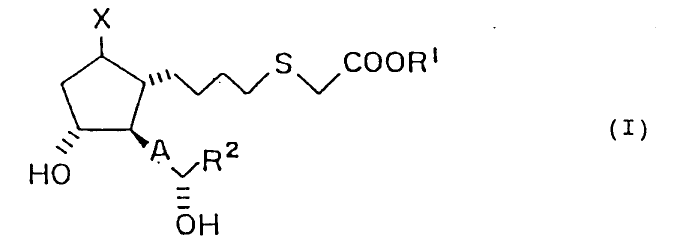 wherein R¹ is a hydrogen atom, an alkyl group having 1 to 10 carbon atoms or a cycloalkyl group having 3 to 10 carbon atoms, and R² is a cycloalkyl group having 3 to 10 carbon atoms, a cycloalkyl group having 3 to 10 carbon atoms substituted by a methyl group, an alkyl group having 1 to 3 carbon atoms substituted by a cycloalkyl group having 3 to 10 carbon atoms, a branched alkyl group having 5 to 10 carbon atoms or a branched alkenyl group having 5 to 10 carbon atoms, X is a halogen atom substituted at the α- or β-configuration, and A is an ethylene group, a vinylene group or an ethynylene group, and salts thereof.

The present invention is to provide the above-mentioned novel PG derivatives and salts thereof for use as an effective ingredient of a pharmaceutical composition.

Further, the present invention is to provide a pharmaceutical composition for lowering intraocular pressure containing the above-mentioned novel PG derivative or a salt thereof as an effective ingredient.

Furthermore, the present invention is to provide use of the above-mentioned novel PG derivative or a salt thereof for the manufacture of a pharmaceutical composition for lowering intraocular pressure.

Still furthermore, the present invention is to provide a method for lowering intraocular pressure comprising administering an effective amount of the above-mentioned PG derivative or a salt thereof to the eye of human.

### BRIEF DESCRIPTION OF THE DRAWINGS

Fig. 1 is a graph which shows the change with time in the intraocular pressure values of rabbits to which an ophthalmic solution containing the compound of the present invention at the concentration of 10⁻⁶ mol/50 µl was applied.

Fig. 2 is a graph which shows the change with time in the intraocular pressure values of rabbits to which an ophthalmic solution containing the compound of the present invention at the concentration of 10⁻⁷ mol/50 µl was applied.

Fig. 3 is a graph which shows the change with time in the intraocular pressure values of rabbits to which an ophthalmic solution containing the compound of the present invention at the concentration of 10⁻⁸ mol/50 µl was applied.

### BEST MODE FOR CARRYING OUT THE INVENTION

In the present invention, the alkyl group having 1 to 10 carbon atoms refers to a straight or branched alkyl group such as, for example, a methyl group, an ethyl group, a propyl group, an isopropyl group, an n-butyl group, an isobutyl group, a tert-butyl group, an n-pentyl group or an isopentyl group. Examples of the cycloalkyl group having 3 to 10 carbon atoms optionally substituted by a methyl group are a cyclopentyl group, a cyclohexyl group, a 2-methylcyclohexyl group and a cycloheptyl group, examples of the alkyl group having 1 to 3 carbon atoms substituted by the cycloalkyl group having 3 to 10 carbon atoms are a cyclopentylmethyl group, a cyclohexylmethyl group, a cyclopentylethyl group, a cyclohexylethyl group, a cycloheptylethyl group and a cyclohexylpropyl group, examples of the branched alkyl group having 5 to 10 carbon atoms are a 1-methylhexyl group and a 2-methylhexyl group, and an example of the branched alkenyl group having 5 to 10 carbon atoms is a 2,6-dimethylhept-5-enyl group.

Examples of the halogen atom are a fluorine atom, a chlorine atom and a bromine atom.

The salt of the compound of Formula (I) refers to a pharmaceutically acceptable salt of the compound of Formula (I) wherein R¹ is a hydrogen atom such as, for example, salts with metals (e.g. sodium, potassium, calcium, magnesium or aluminum) or salts with organic amines (e.g. a trialkylamine).

In the compound of Formula (I) of the present invention, R¹ is preferably a hydrogen atom or an alkyl group having 1 to 6 carbon atoms, and especially a hydrogen atom, a methyl group, an isopropyl group or a t-butyl group. An preferable example of the cycloalkyl group for R² is a cycloalkyl group having 5 to 8 carbon atoms, and preferable examples of the cycloalkyl group optionally substituted by a methyl group for R² are a cyclopentyl group, a cyclohexyl group, a 2-methylcyclohexyl group and a cycloheptyl group. The alkyl group substituted by the cycloalkyl group for R² is preferably an alkyl group having 1 to 3 carbon atoms substituted by a cycloalkyl group having 5 to 8 carbon atoms, and especially a cyclohexylmethyl group or a cyclopentylmethyl group. A is preferably an ethynylene group.

Preferable compounds of the present invention are as follows.
3-Thia-9-deoxy-9β-chloro-16,17,18,19,20-pentanor-15-cyclohexyl-13,14-didehydro-PGF₁α
3-Thia-9-deoxy-9β-chloro-16,17,18,19,20-pentanor-15-cyclohexyl-13,14-didehydro-PGF₁α isopropyl ester
3-Thia-9-deoxy-9β-chloro-16,17,18,19,20-pentanor-15-cycloheptyl-13,14-didehydro-PGF₁α
3-Thia-9-deoxy-9β-bromo-16,17,18,19,20-pentanor-15-cyclohexyl-13,14-didehydro-PGF₁α methyl ester
3-Thia-9-deoxy-9β-bromo-16,17,18,19,20-pentanor-15-cyclohexyl-13,14-didehydro-PGF₁α
3-Thia-9-deoxy-9-fluoro-16,17,18,19,20-pentanor-15-cyclohexyl-13,14-didehydro-PGF₁

The compounds of the present invention can be prepared, for example, by the following methods.

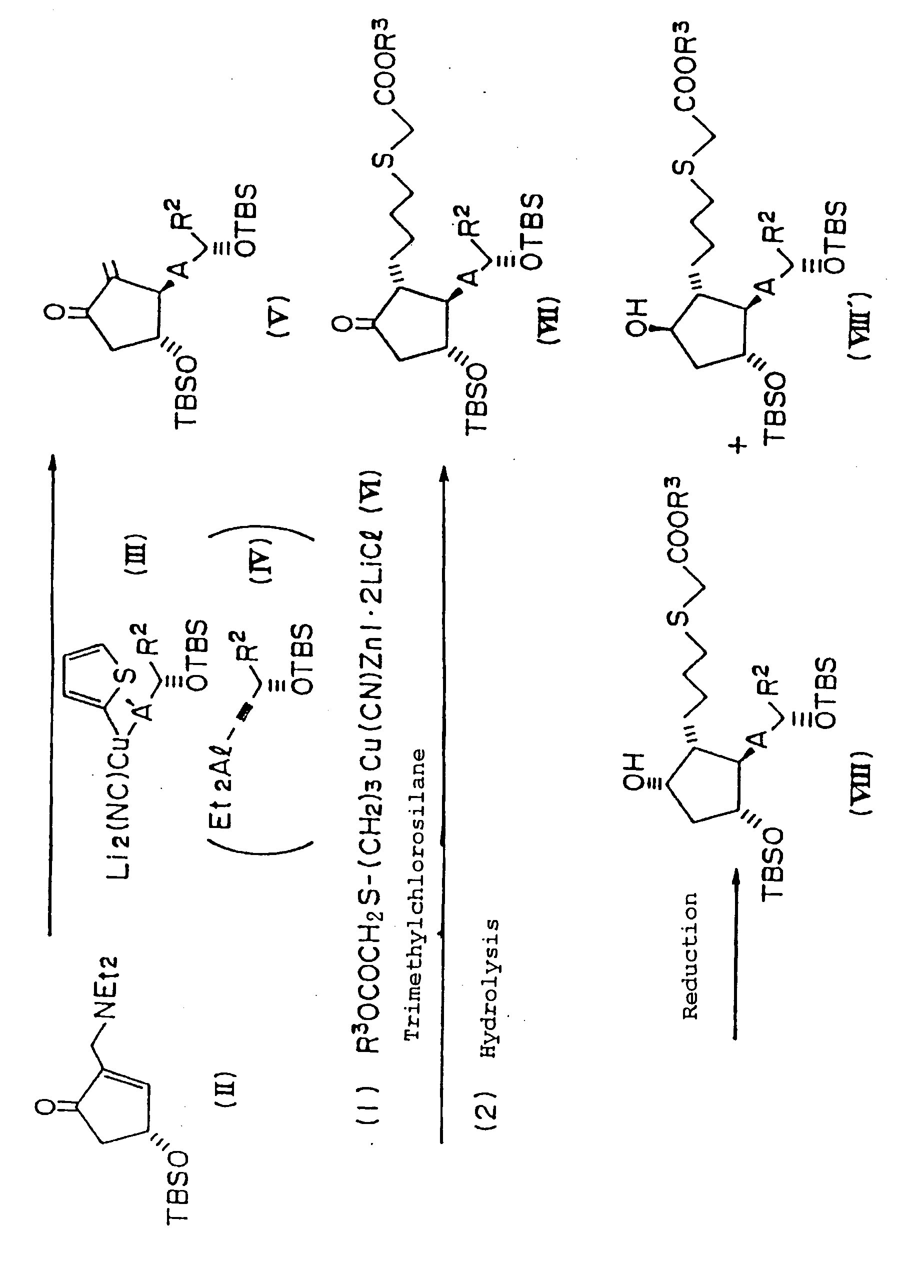

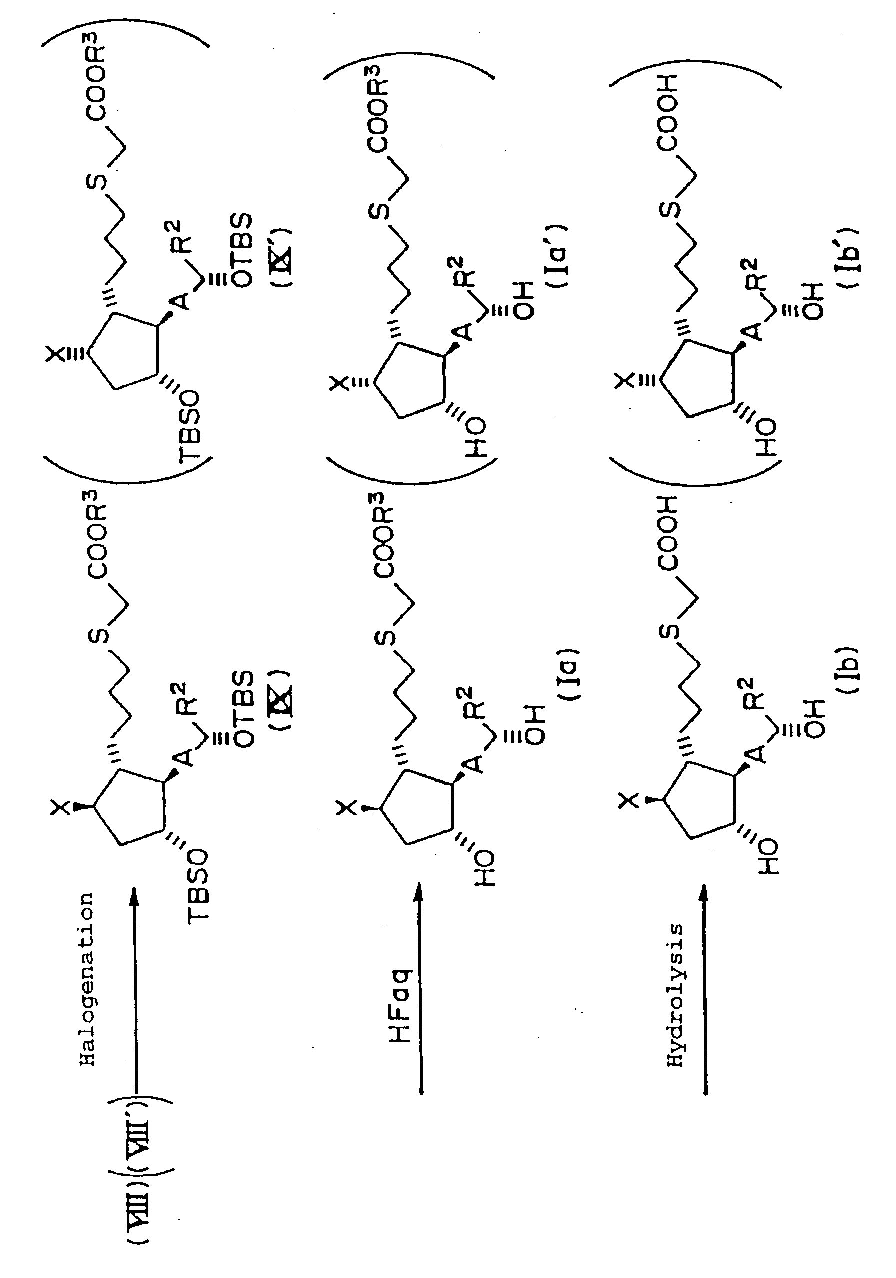

In the reaction scheme, TBS is a t-butyldimethylsilyl group, R³ is an alkyl group having 1 to 10 carbon atoms or a cycloalkyl group having 3 to 10 carbon atoms, A' is an ethylene group or a vinylene group, Et is an ethyl group, and A, R² and X are as defined in Formula (I).
① Firstly, a known compound of Formula (II) is reacted with 0.8 to 2.0 equivalents of a compound of Formula (III) or (IV) in an inert solvent at -10 to 30°C according to the method of Sato et al. (Journal of Organic Chemistry, Vol. 53, page 5590 (1988)) to give a compound of Formula (V) stereoselectively. Herein, the compound wherein A is an ethylene group or a vinylene group (i.e. A is A') or the compound wherein A is an ethynylene group can be obtained by using a compound of Formula (III) or a compound of Formula (IV), respectively. Examples of the inert solvent are benzene, toluene, tetrahydrofuran, diethyl ether, methylene chloride, n-hexane and n-pentane.
② The compound of Formula (V) is reacted with 0.5 to 4 equivalents of an organic copper compound of Formula (VI) and 0.5 to 4 equivalents of trimethylchlorosilane in an inert solvent at -78 to 40°C, and then the resulting compound is hydrolyzed with an inorganic or organic acid or an amine salt thereof in an organic solvent at 0 to 40°C to give a compound of Formula (VII) stereoselectively. Examples of the inert solvent are benzene, toluene, tetrahydrofuran, diethyl ether, methylene chloride, n-hexane and n-pentane. Examples of the inorganic acid are hydrochloric acid, sulfuric acid and nitric acid. Examples of the organic acid are acetic acid and p-toluenesulfonic acid. An example of the amine salt is pyridinium p-toluenesulfonate. Examples of the organic solvent are acetone, methanol, ethanol, isopropanol, diethyl ether and a mixture thereof.
③ The compound of Formula (VII) is reduced with 0.5 to 5 equivalents of a reductant such as potassium borohydride, sodium borohydride, lithium triamyl borohydride or lithium tri-sec-butyl borohydride in an organic solvent at -78 to 40°C to give compounds of Formulae (VIII) and (VIII'). Examples of the organic solvent are tetrahydrofuran, diethyl ether, ethyl alcohol and methyl alcohol. These compounds of Formulae (VIII) and (VIII') can be purified by a conventional separation method such as column chromatography.
④ The compound of Formula (VIII) or (VIII') is tosylated with 1 to 6 equivalents of p-toluenesulfonyl chloride in pyridine in the presence of 0.8 to 6 equivalents of 4-dimethylaminopyridine at -20 to 40°C, followed by chlorination with 1 to 6 equivalents of tetra-n-butylammonium chloride to give a compound of Formula (IX) or (IX') wherein X is a chlorine atom, respectively. Herein, bromination or fluorination can be also carried out in an ordinary manner. For example, bromination can be carried out by a reaction with 1 to 10 equivalents of carbon tetrabromide in the presence of 1 to 10 equivalents of triphenylphosphin and 1 to 10 equivalents of pyridine in acetonitrile, and fluorination can be carried out by a reaction with 5 to 20 equivalents of diethylaminosulfur trifluoride (DAST) in methylene chloride.
⑤ The protective group of the hydroxyl group of compound of Formula (IX) or (IX') is removed with hydrofluoric acid or pyridinium poly(hydrogenfluoride) under the conventional conditions to give a compound of Formula (I) wherein R¹ is other than a hydrogen atom, i.e. a PG derivative of Formula (Ia) or (Ia') of the present invention.
⑥ The compound of Formula (I) wherein R¹ is a hydrogen atom, i.e. a PG derivative of Formula (Ib) or (Ib') of the present invention can be obtained by hydrolyzing the ester moiety of the PG derivative of Formula (Ia) or (Ia') with 1 to 6 equivalents of a base in a solvent ordinarily used for hydrolysis. Examples of the base are lithium hydroxide and potassium carbonate. Examples of the solvent are acetonitrile, acetone, methanol, ethanol, water and a mixture thereof.

The PG derivatives and salts thereof of the present invention can be applied in the form of ophthalmic preparations such as sterile aqueous solution, aqueous suspensions, non-aqueous solutions or non-aqueous suspensions. A diluent to be used for the aqueous solutions or suspensions includes distilled water or physiological saline. A diluent to be used for the non-aqueous solution or suspensions includes vegetable oils, liquid paraffin, mineral oils, propylene glycol or p-octyldodecanol. Also contained in the preparations may be components such as isotonic agents for making the same osmotic pressure as that of tears (e.g. sodium chloride or potassium chloride), buffer solutions (e.g. borate buffer, phosphate buffer, citrate buffer or carbonate buffer), stabilizers (e.g. sodium sulfite or EDTA), thickening agents (e.g. glycerin or carboxymethyl cellulose), preservatives (e.g. parabens, benzalkonium chloride or sorbin), solubilizers (polysorbate 80, polyoxyethylene hardened caster oil or propylene glycol) or pH regulators. These components can be used after asepsis by sterilization filtration.

When applied in the form of ophthalmic ointments, bases (e.g. vaseline, selen 50, plastibase or maclogols), surfactants (e.g. polysorbate or purified lanoline) and jellies (e.g. carboxymethyl cellulose, methylcellulose or carboxyvinyl polymer) can be also contained therein.

The compounds of the present invention can be used as therapeutical agents of glaucoma because of having a lowering action of intraocular pressure. In this case, conventional choline-type ocular hypotensive agents, miotic agents (e.g. pilocarpine), hyperostotic agents for intravenous administration (e.g. mannitol), antiseptic agents for ophthalmology (e.g. parabens or chlorobutanol) or preventional or therapeutical agents of inflammation (e.g. penicillin or sulfonamide) can be also contained.

As apparent from the following experiments, the compounds of the present invention have a potent lowering action of intraocular pressure with little side-effects, and therefore are effective for the treatment of glaucoma and other diseases and symptoms which are required to lower intraocular pressure.

The lowering action of the PG derivatives of the present invention is illustrated in more detail by the following experiments.

### Experiment 1 Lowering Action of Rabbit Intraocular Pressure Method.

Rabbit intraocular pressure was determined according to the method of Goh et al (British Journal of Ophthalmology, vol. 72, page 461, 1988). Rabbits (white rabbits, weighing 2.0 to 2.5 kg) were fixed in holders for oral administration, and corneal surface anesthetization was provided by instillation of one or two drops of a xylocaine solution (made by Fujisawa Pharmaceutical Co.) for ophthalmology. Subsequently, intraocular pressure (IOP) was measured by using an electrotonometer (made by Alcon Inc.) from the gas pressure given by a sensor connected to a silicone rubber plate attached to rabbit cornea. The measurement was repeated 3 times at intervals of 15 minutes, and the medium value of the intraocular pressures of both eyes serves as a control.

50 µl of the compound of the present invention dissolved in a vehicle (a physiological saline containing 10% ethanol) (10⁻⁸, 10⁻⁷ and R⁻⁶ mol/50 µl) was topically administered to the left eye of each rabbit, and 50 µl of the vehicle only was similarly administered to the right eye. After the administration, intraocular pressure was measured at 0.5, 1, 2 and 3 hours. The compound of the present invention used herein is that of Formula (I) wherein R¹ is a hydrogen atom, R² is a cyclohexyl group, X is a β-chlorine atom and A is an ethynylene group, e.g. a compound of Formula (X).

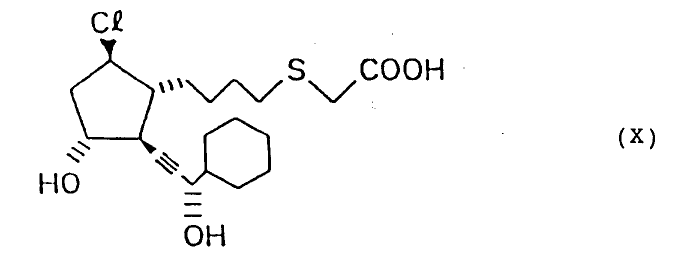

Statistical analyses was conducted by comparison of the intraocular pressure value before administration and the intraocular pressure values at the designated times after the administration according to paired T-test. Significant level is 5% of two-sided risk rate, and p < 5% represents a significant difference. Four rabbits were used for each group.

### Results

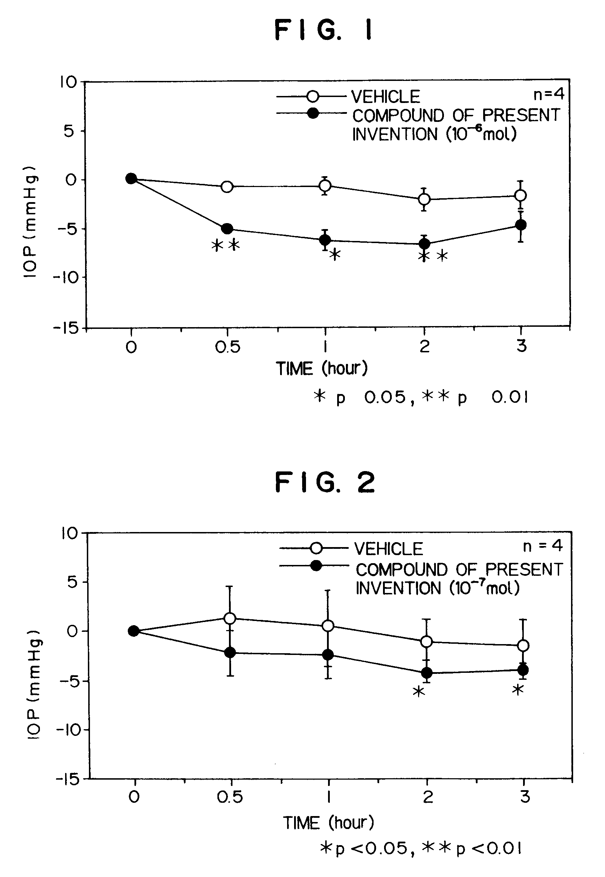

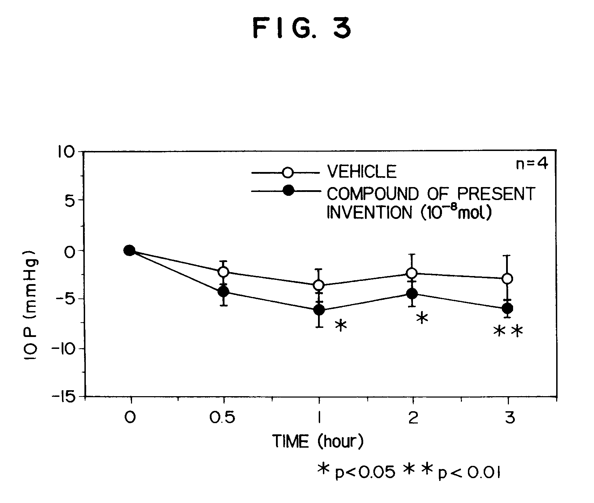

The changes with time in the intraocular pressure values of rabbits to which the ophthalmological solutions of the compound at the desired concentrations (10⁻⁸, 10⁻⁷ and 10⁻⁶ mol/50 µl) were administered are shown in Figs. 1 to 3.

By the administration of the ophthalmic solution containing the compound of the present invention, intraocular pressure was observed to lower promptly and then to lower significantly at 2 or 3 hours after the administration. In cases of 10⁻⁸ and 10⁻⁷ mol/50 µl, stable lowering of intraocular pressure was observed without any side-effects. Furthermore, elevation of intraocular pressure prior to the lowering of intraocular pressure was not observed in the groups administered at any concentrations. On the other hand, there was observed no significant change in another eye to which the vehicle was administered when compared with the value prior to administration.

In each experiment, side-effects are particularly illustrated as follows.
Irritation: There was observed ordinary status without abnormality in each experiment.
Miosis: Miosis was not found in each experiment.
Congestion of iris: The iris was congested reddishly all around in case of 10⁻⁶ mol/50 µl (Fig. 1), but not congested in cases of 10⁻⁸ and 10⁻⁷ mol/50 µl.
Congestion of conjunctivas: No congestion was found in each experiment.

### Experiments 2 to 6

According to a procedure similar to that of Experiment 1, 50 µl of the compounds of the present invention (10⁻⁸ mol/50 µl) was topically administered to each left eye of four rabbits for each group, 50 µl of the vehicle (a physiological saline solution containing 10% ethanol) was also topically administered to the right eye, and the intraocular pressure was determined at 3 hours after the administration. The lowering action of intraocular pressure of the left eye against the right eye is as follows. Herein, for example, Examples 2(4) and 7(5) mean the compounds obtained in Examples 2(4) and 7(5), respectively.

| | | |
|---|---|---|
| Experiment 2 | Example 2(4) | -5.3 mmHg |
| Experiment 3 | Example 7(5) | -2.3 mmHg |
| Experiment 4 | Example 13(2) | -2.4 mmHg |
| Experiment 5 | Example 13(3) | -2.8 mmHg |
| Experiment 6 | Example 22(3) | -3.8 mmHg |

In each experiment, side-effects were little found.

The present invention is illustrated in more detail by the following examples and experiments. The compounds obtained in the examples are shown in Tables 1 and 2, wherein A, X, R² and R¹ are as defined in Formula (I).

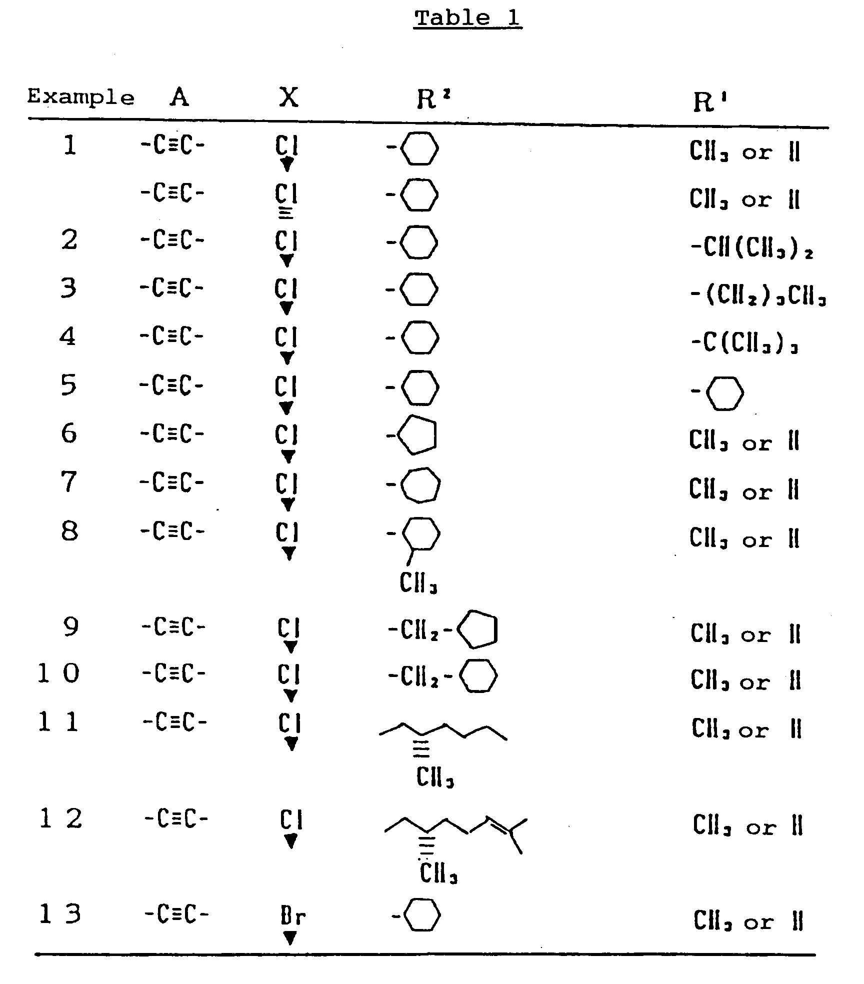

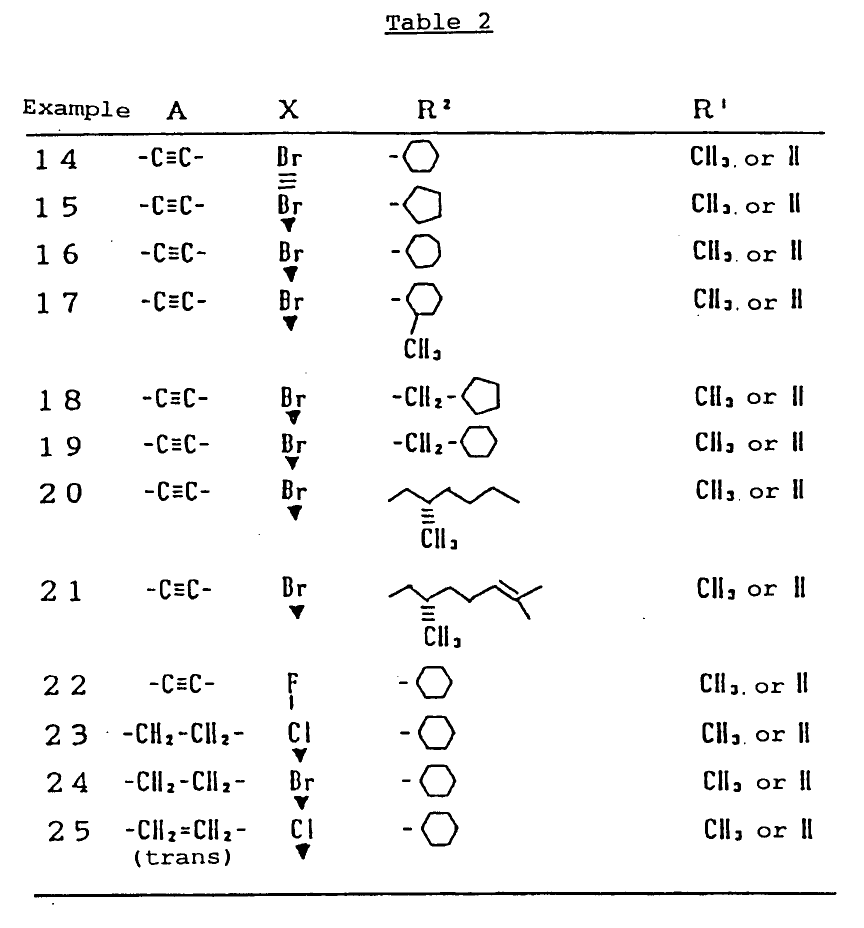

### Example 1

### Preparations of 3-thia-9-deoxy-9β-chloro-16,17,18,19,20-pentanor-15-cyclohexyl-13,14-didehydro-PGF₁α (the compound used in Experiment 1), 3-thia-9-deoxy-9α-chloro-16,17,18,19,20-pentanor-15-cyclohexyl-13,14-didehydro-PGF₁α and methyl esters thereof

(1) (3S)-3-t-(Butyldimethylsiloxy)-3-cyclohexylprop-1-yne (3.61 g) was dissolved in benzene (28.8 ml), and n-butyl lithium (1.95 M, n-hexane solution, 6.4 ml) was added thereto at 0°C, followed by stirring at the same temperature for 30 minutes. To the solution was added diethyl aluminum chloride (0.97 M, hexane solution, 14.8 ml) at 0°C, followed by stirring at room temperature for 30 minutes. (4R)-2-(N,N-Diethylamino)methyl-4-(t-butyldimethylsiloxy)cyclopent-2-en-1-one (0.25 M, hexane solution, 14.8 ml) was added to the solution at room temperature, followed by stirring for 15 minutes. The reaction solution was poured into a mixture of hexane (100 ml), a saturated aqueous ammonium chloride solution (100 ml) and an aqueous hydrochloric acid solution (3M, 30 ml) with stirring, and the organic layer was separated and washed with a saturated aqueous sodium bicarbonate solution (50 ml). The resulting organic layer was dried and concentrated, and the resulting crude product was purified by silica gel column chromatography (eluent; hexane : ether = 10:1) to give 3.69 g of (3R,4R)-2-methylene-3-[(3'S)-3'-(t-butyldimethylsiloxy)-3'-cyclohexylprop-1'-ynyl]-4-(t-butyldimethylsiloxy)cyclopentan-1-one.
   ¹H-NMR(CDCl₃, 200 MHz) δ ppm; 0.07, 0.08 and 0.12(3s, 12H), 0.88(s, 18H), 0.92-1.92(m, 11H), 2.32(dd, J=17.8, 7.4Hz, 1H), 2.71(dd, J=17.8, 6.5Hz, 1H), 3.48-3.58(m, 1H), 4.11(dd, J=6.2, 1.4Hz, 1H), 4.20-4.32(m, 1H), 5.55(d, J=2.6Hz, 1H), 6.13(d, J=3.0Hz, 1H).
   IR(neat):
      2930, 2850, 1375, 1640, 1470, 1380, 1255, 1105, 830, 770 cm⁻¹.
(2) Copper (I) cyanide-lithium dichloride (1.0 M, tetrahydrofuran solution, 16.9 ml, 16.9 mmol) was added to 4-thia-5-carbomethoxypentylzinc (II) iodide (0.75 M, tetrahydrofuran solution, 18.0 ml, 13.5 mmol) under an argon stream at -70°C, followed by stirring at the same temperature for 20 minutes. To the solution were added a solution of (3R,4R)-2-methylene-3-[(3'S)-3'-(t-butyldimethylsiloxy)-3'-cyclohexylprop-1'-ynyl]-4-(t-butyldimethylsiloxy)cyclopentan-1-one (3.21 g, 6.74 mmol) in 23.6 ml of diethyl ether and chlorotrimethylsilane (1.54 ml, 12.1 mmol) at -70°C, and the temperature was elevated to 0°C with stirring over about an hour period. To the reaction solution was added 100 ml of a saturated aqueous ammonium chloride solution, followed by extracting with n-hexane. The organic layer was washed with a saturated aqueous sodium bicarbonate solution and a saturated aqueous sodium chloride solution, dried and concentrated. The resulting residue was dissolved in diethyl ether (6.8 ml) - isopropyl alcohol (27 ml), and then pyridinium p-toluenesulfonate (85 mg, 0.34 mmol) was added thereto, followed by stirring at room temperature for 12 hours. The reaction solution, after addition of 200 ml of n-hexane, was washed with a saturated aqueous sodium bicarbonate solution and a saturated aqueous sodium chloride solution, dried and concentrated. The resulting crude product was purified by silica gel column chromatography (eluent; n-hexane : AcOEt = 15:1) to give 2.51 g (4.02 mmol) of 3-thia-16,17,18,19,20-pentanor-15-cyclohexyl-13,14-didehydro-PGE₁ methyl ester 11,15-bis(t-butyldimethylsilyl ether).
   ¹H-NMR(CDCl₃, 200MHz) δ ppm; 0.08(s, 3H), 0.09(s, 3H), 0.10(s, 3H), 0.12(s, 3H), 0.84-1.92(m, 17H), 0.89(s, 9H), 0.90(s, 9H), 2.03-2.27(m, 1H), 2.17(dd, J=18.2, 7.0Hz, 1H), 2.56-2.76(m, 4H), 3.22(s, 2H), 3.74(s, 3H), 4.09(dd, J=6.2, 1.5Hz, 1H), 4.23-4.35(m, 1H).
   IR(neat):
      2929, 2856, 2235, 1746, 1463, 1436, 1362, 1280, 1257, 1133, 1103, 1008, 899, 838, 779, 670 cm⁻¹.
(3) A solution of the compound obtained in (2) (1.55 g, 2.47 mmol) in methyl alcohol (24.7 ml) was cooled to 0°C, and then potassium borohydride (0.276 g, 4.94 mmol) was added thereto, followed by stirring for 15 minutes. After addition of water, the mixture was extracted with ether-n-hexane (2:1, 210 ml), and the extract was washed with a saturated aqueous ammonium chloride solution and a saturated aqueous sodium chloride solution, dried and concentrated. The resulting crude product was purified by silica gel column chromatography (eluent; n-hexane : AcOEt = 7:1 - 4:1) to give 608 mg (0.970 mmol) of 3-thia-16,17,18,19,20-pentanor-15-cyclohexyl-13,14-didehydro-PGF₁α methyl ester 11,15-bis(t-butyldimethylsilyl ether) and 642 mg (1.024 mmol) of 3-thia-16,17,18,19,20-pentanor-15-cyclohexyl-13,14-didehydro-PGF₁β-methyl ester 11,15-bis(t-butyldimethylsilyl ether).
   3-Thia-16,17,18,19,20-pentanor-15-cyclohexyl-13,14-didehydro-PGF₁α methyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.11(s, 3H), 0.84-1.92(m, 19H), 0.88(s, 9H), 0.90(s, 9H), 1.93-2.07(m, 1H), 2.40-2.50(m, 1H), 2.53(br s, 1H), 2.65(t, J=7.0Hz, 2H), 3.23(s, 2H), 3.74(s, 3H), 4.01-4.19(m, 1H), 4.07(dd, J=6.3, 1.8Hz, 1H), 4.23-4.32(m, 1H).
   IR(neat);
      3523, 2929, 2856, 2231, 1740, 1472, 1463, 1437, 1408, 1389, 1362, 1338, 1279, 1256, 1217, 1133, 1103, 1071, 1007, 964, 939, 899, 838, 778, 669cm⁻¹.

   3-Thia-16,17,18,19,20-pentanor-15-cyclohexyl-13,14-didehydro-PGF₁β methyl ester 11,15-bis(t-butyldimethylsilyl ether)
   ¹H-NMR(CDCl₃, 200MHz) δ ppm; 0.07(s, 3H), 0.08(s, 6H), 0.11(s, 3H), 0.83-1.94(m, 20H), 0.88(s, 9H), 0.90(s, 9H), 2.23(ddd, J=9.0, 6.2, 1.6Hz, 1H), 2.65(t, J=7.0Hz, 2H), 3.22(s, 2H), 3.74(s, 3H), 3.90-4.06(m, 1H), 4.08(dd, J=6.2, 1.6Hz, 1H), 4.16-4.30(m, 1H).
   IR(neat):
      3458, 2928, 2856, 2232, 1739, 1472, 1463, 1451, 1388, 1361, 1338, 1281, 1255, 1132, 1104, 1068, 1007, 898, 838, 778, 670cm⁻¹.
(4) Methanesulfonyl chloride (0.31 ml, 4.0 mmol) was added to a solution of 3-thia-16,17,18,19,20-pentanor-15-cyclohexyl-13,14-didehydro-PGF₁α methyl ester 11,15-bis(t-butyldimethylsilyl ether) obtained in (3) (839 mg, 1.34 mmol) in pyridine (6.70 ml) at 0°C under an argon stream, followed by stirring at room temperature for 2 hours. To the solution was added a toluene solution (6.70 ml) of tetra-n-butylammonium chloride (7.45 g, 26.8 mmol), followed by stirring at 45°C for 4 hours. After addition of water, the mixture was extracted with ether, and the extract was washed with a saturated aqueous sodium chloride solution, dried and concentrated. The resulting crude product was purified by silica gel column chromatography (eluent; n-hexane : AcOEt = 10:1) to give 764 mg (1.18 mmol) of 3-thia-9-deoxy-9β-chloro-16,17,18,19,20-pentanor-15-cyclohexyl-13,14-didehydro-PGF₁α-methyl ester 11,15-bis(t-butyldimethylsilyl ether).
   ¹H-NMR(CDCl₃, 200MHz) δ ppm; 0.07(s, 3H), 0.08(s, 6H), 0.11(s, 3H), 0.74-1.92(m, 17H), 0.88(s, 9H), 0.90(s, 9H), 1.98-2.20(m, 2H), 2.14(dd, J=7.7, 5.7Hz, 1H), 2.28(ddd, J=8.9, 5.1, 1.6Hz, 1H), 2.58-2.72(m, 2H), 3.23(s, 2H), 3.74(s, 3H), 3.95(dd, J=15.5, 7.7Hz, 1H), 4.08(dd, J=6.1, 1.6Hz, 1H), 4.25(q, J=5.1Hz, 1H).
   IR(neat):
      2929, 2856, 2233, 1741, 1472, 1463, 1451, 1408, 1389, 1362, 1339, 1279, 1256, 1189, 1133, 1101, 1073, 1007, 962, 939, 899, 838, 815, 778, 670, 588cm⁻¹.

   Following a manner similar to as described above using 3-thia-16,17,18,19,20-pentanor-15-cyclohexyl-13,14-didehydro-PGF₁β methyl ester 11,15-bis(t-butyldimethylsilyl ether) obtained in (3), 3-thia-9-deoxy-9α-chloro-16,17,18,19,20-pentanor-15-cyclohexyl-13,14-didehydro-PGF₁α ester 11,15-bis(t-butyldimethylsilyl ether) was obtained.
   ¹H-NMR(CDCl₃, 200MHz) δ ppm; 0.06(s, 3H), 0.08(s, 3H), 0.09(s, 3H), 0.11(s, 3H), 0.71-2.18(m, 18H), 0.89(s, 9H), 0.90(s, 9H), 2.02(ddd, J=15.3, 3.6, 1.7Hz, 1H), 2.46-2.76(m, 4H), 3.23(s, 2H), 3.74(s, 3H), 4.04-4.27(m, 2H), 4.27-4.36(m, 1H).
   IR(neat):
      2929, 2856, 2237, 1741, 1472, 1463, 1451, 1387, 1361, 1279, 1256, 1102, 1073, 1007, 888, 838, 778, 670, 626cm⁻¹.
(5) An aqueous hydrofluoric acid solution (6.0 ml) was added to a solution of 3-thia-9-deoxy-9β-chloro-16,17,18,19,20-pentanor-15-cyclohexyl-13,14-didehydro-PGF₁α methyl ester 11,15-bis(t-butyldimethylsilyl ether) obtained in (4) (738 mg, 1.14 mmol) in tetrahydrofuran (20 ml) at 0°C, followed by stirring for 6 hours. The reaction solution was poured into ethyl acetate (60 ml) - a saturated aqueous sodium bicarbonate solution (170 ml) with stirring, and the aqueous layer was extracted with ethyl acetate. The resulting organic layer was dried and concentrated, and the resulting crude product was purified by silica gel column chromatography (eluent; n-hexane : AcOEt = 2:1) to give 474 mg (1.14 mmol) of 3-thia-9-deoxy-9β-chloro-16,17,18,19,20-pentanor-15-cyclohexyl-13,14-didehydro-PGF₁α methyl ester.
   ¹H-NMR(CDCl₃, 300MHz) δ ppm; 0.96-1.35(m, 6H), 1.46-1.90(m, 11H), 2.09-2.21(m, 1H), 2.19(ddd, J=14.1, 7.6, 6.1Hz, 1H), 2.23-2.32(m, 1H), 2.31(ddd, J=9.9, 6.4, 1.8Hz, 1H), 2.58-2.73(m, 2H), 3.24(s, 2H), 3.75(s, 3H), 3.95(dd, J=14.7, 7.6Hz, 1H), 4.17(dd, J=6.0, 1.8Hz, 1H), 4.33-4.41(m, 1H).
   IR(KBr):
      3392, 2926, 2854, 2236, 1736, 1457, 1439, 1410, 1370, 1341, 1278, 1228, 1140, 1126, 1097, 1016, 892, 730, 706, 546cm⁻¹.

   Following a manner similar as described above using 3-thia-9-deoxy-9α-chloro-16,17,18,19,20-pentanor-15-cyclohexyl-13,14-didehydro-PGF₁α methyl ester 11,15-bis(t-butyldimethylsilyl ether) obtained in (4), 3-thia-9-deoxy-9α-chloro-16,17,18,19,20-pentanor-15-cyclohexyl-13,14-didehydro-PGF₁α methyl ester was obtained.
   ¹H-NMR(CDCl₃, 300MHz) δ ppm; 0.97-2.00(m, 18H), 2.15(ddd, J=15.7, 3.0, 1.1Hz, 1H), 2.54-2.78(m, 3H), 2.73(ddd, J=11.5, 6.2, 2.0Hz, 1H), 3.23(s, 2H), 3.74(s, 3H), 4.17(dd, J=6.0, 1.8Hz, 1H), 4.27(ddd, J=8.6, 6.1, 3.0Hz, 1H), 4.35-4.43(m, 1H).
   IR(neat):
      3401, 2927, 2854, 2235, 1736, 1437, 1281, 1137, 1085, 1011, 894, 834, 623cm⁻¹.
(6) Lithium hydroxide monohydride (122 mg, 17.7 mmol) was added to a solution of 3-thia-9-deoxy-9β-chloro-16,17,18,19,20-pentanor-15-cyclohexyl-13,14-didehydro-PGF₁α methyl ester obtained in (5) (222 mg, 0.532 mmol) in methanol (17.7 ml) - water (1.8 ml), followed by stirring at room temperature for 2 hours. The mixture was neutralized by adding 1N aqueous hydrochloric acid solution, and concentrated. To the residue were added 0.1 N hydrochloric acid (4 ml), ether (40 ml) and AcOEt (8 ml), followed by salting out with ammonium sulfate. The organic layer was collected by fractionation, and purified by silica gel column chromatography (AcOEt elution) to give 171 mg (0.424 mmol) of 3-thia-9-deoxy-9β-chloro-16,17,18,19,20-pentanor-15-cyclohexyl-13,14-didehydro-PGF₁α as colorless crystals (the compound used in Experiment).
   ¹H-NMR(CDCl₃, 300MHz) δ ppm; 0.96-1.34(m, 6H), 1.47-1.89(m, 11H), 2.09-2.33(m, 2H), 2.20(ddd, J=14.1, 7.6, 6.5Hz, 1H), 2.33(ddd, J=9.9, 6.5, 1.8Hz, 1H), 2.64(brs, 2H), 2.65-2.75(m, 2H), 3.23(s, 2H), 3.95(dd, J=14.5, 7.6Hz, 1H), 4.21(dd, J=6.0, 1.8Hz, 1H), 4.37(q, J=6.5Hz, 1H).
   IR(neat):
      3369, 2928, 2854, 2236, 1713, 1451, 1283, 1140, 1084, 1008, 894, 758, 668cm⁻¹.

   Following a manner similar to as described above using 3-thia-9-deoxy-9α-chloro-16,17,18,19,20-pentanor-15-cyclohexyl-13,14-didehydro-PGF₁α methyl ester obtained in (5), 3-thia-9-deoxy-9α-chloro-16,17,18,19,20-pentanor-15-cyclohexyl-13,14-didehydro-PGF₁α was obtained.
   ¹H-NMR(CDCl₃, 300MHz) δ ppm; 0.83-2.08(m, 18H), 2.16(ddd, J=15.6, 2.9, 1.1Hz, 1H), 2.57-2.81(m, 4H), 2.84-3.60(m, 5H), 4.23(dd, J=6.2, 2.0Hz, 1H), 4.28(ddd, J=8.7, 6.0, 2.8Hz, 1H), 4.39(t, J=4.8Hz, 1H).
   IR(KBr):
      3392, 2932, 2856, 2240, 1705, 1458, 1416, 1345, 1304, 1278, 1212, 1140, 1108, 1085, 1063, 1042, 1002, 890, 843, 723, 682, 620cm⁻¹.

### Example 2

### Preparation of 3-thia-9-deoxy-9β-chloro-16,17,18,19,20-pentanor-15-cyclohexyl-13,14-didehydro-PGF₁α isopropyl ester

(1) Following a manner similar to that of Example 1(2) using the compound obtained in Example 1(1) and 4-thia-5-carboisopropoxypentyl zinc (II) iodide, 3-thia-16,17,18,19,20-pentanor-15-cyclohexyl-13,14-didehydro-PGE₁ isopropyl ester 11,15-bis(t-butyldimethylsilyl ether) was obtained.
   ¹H-NMR(CDCl₃, 200MHz) δ ppm; 0.08(s, 3H), 0.09(s, 3H), 0.10(s, 3H), 0.12(s, 3H), 0.83-1.94(m, 17H), 0.89(s, 9H), 0.90(s, 9H), 1.28(d, J=6.3Hz, 6H), 2.03-2.28(m, 1H), 2.17(dd, J=18.9, 6.8Hz, 1H), 2.51-2.88(m, 4H), 3.19(s, 2H), 4.09(dd, J=6.2, 1.3Hz, 1H), 4.22-4.36(m, 1H), 5.05(sept, J=6.3Hz, 1H).
   IR(neat):
      2980, 2930, 2856, 2235, 1730, 1471, 1411, 1387, 1375, 1277, 1214, 1107, 1007, 965, 898, 838, 779, 670cm⁻¹.
(2) Following a manner similar to that of Example 1(3) using the compound obtained in (1), 3-thia-16,17,18,19,20-pentanor-15-cyclohexyl-13,14-didehydro-PGF₁α isopropyl ester 11,15-bis(t-butyldimethylsilyl ether) and 3-thia-16,17,18,19,20-pentanor-15-cyclohexyl-13,14-didehydro-PGF₁β isopropyl ester 11,15-bis(t-butyldimethylsilyl ether) was obtained.
   3-Thia-16,17,18,19,20-pentanor-15-cyclohexyl-13,14-didehydro-PGF₁α isopropyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.11(s, 3H), 0.74-2.12(m, 20H), 0.88(s, 9H), 0.90(s, 9H), 1.26(d, J=6.3Hz, 6H), 2.39-2.51(m, 1H), 2.65(t, J=7.1Hz, 2H), 3.18(s, 2H), 4.04-4.17(m, 1H), 4.07(dd, J=6.3, 1.9Hz, 1H), 4.22-4.33(m, 1H), 5.04(sept, J=6.3Hz, 1H).
   IR(neat);
      3464, 2929, 2856, 2232, 1729, 1472, 1388, 1277, 1256, 1106, 1071, 1006, 965, 899, 838, 778, 669cm⁻¹.

   3-Thia-16,17,18,19,20-pentanor-15-cyclohexyl-13,14-didehydro-PGF₁β isopropyl ester 11,15-bis(t-butyldimethylsilyl ether)
   ¹H-NMR(CDCl₃, 200MHz) δ ppm; 0.07(s, 3H), 0.08(s, 6H), 0.11(s, 3H), 0.78-2.00(m, 18H), 0.88(s, 9H), 0.90(s, 9H), 1.26(d, J=6.3Hz, 6H), 1.88(t, J=6.3Hz, 2H), 2.23(ddd, J=9.3, 6.3, 1.6Hz, 1H), 2.64(t, J=7.0Hz, 2H), 3.17(s, 2H), 3.98(q, J=6.3Hz, 1H), 4.07(dd, J=6.3, 1.6Hz, 1H), 4.22(q, J=6.3Hz, 1H), 5.04(sept, J=6.3Hz, 1H).
   IR(neat):
      3435, 2929, 2856, 2233, 1729, 1472, 1387, 1375, 1361, 1279, 1255, 1107, 1071, 1006, 965, 898, 838, 778, 669cm⁻¹.
(3) Following a manner similar to that of Example 1(4) using 3-thia-16,17,18,19,20-pentanor-15-cyclohexyl-13,14-didehydro-PGF₁α isopropyl ester 11,15-bis(t-butyldimethylsilyl ether) obtained in (2), 3-thia-9-deoxy-9β-chloro-16,17,18,19,20-pentanor-15-cyclohexyl-13,14-didehydro-PGF₁α isopropyl ester 11,15-bis(t-butyldimethylsilyl ether) was obtained.
   ¹H-NMR(CDCl₃, 200MHz) δ ppm; 0.07(s, 3H), 0.08(s, 6H), 0.11(s, 3H), 0.74-2.20(m, 20H), 0.88(s, 9H), 0.90(s, 9H), 2.17(d, J=6.3Hz, 6H), 2.28(ddd,J=7.7, 4.8, 1.7Hz, 1H), 2.57-2.71(m, 2H), 3.18(s, 2H), 3.95(dd, J=15.5, 7.6Hz, 1H), 4.07(dd, J=6.2, 1.7Hz, 1H), 4.18-4.30(m, 1H), 5.04(sept, J=6.3Hz, 1H).
   IR(neat):
      2929, 2856, 2231, 1729, 1472, 1386, 1362, 1276, 1255, 1107, 1072, 1006, 964, 898, 837, 778, 669cm⁻¹.
(4) Following a manner similar to that of Example 1(5) using the compound obtained in (3), 3-thia-9-deoxy-9β-chloro-16,17,18,19,20-pentanor-15-cyclohexyl-13,14-didehydro-PGF₁α isopropyl ester was obtained.
   ¹H-NMR(CDCl₃, 300MHz) δ ppm; 0.95-1.35(m, 6H), 1.27(d, J=6.3Hz, 6H), 1.46-1.90(m, 11H), 1.95(s, 2H), 2.08-2.34(m, 2H), 2.19(ddd, J=13.9, 7.8, 6.1Hz, 1H), 2.27(dt, J=13.9, 7.0Hz, 1H), 2.61-2.71(m, 2H), 3.18(s, 2H), 3.91-3.99(m, 1H), 4.16(dd, J=6.0, 1.9Hz, 1H), 4.33-4.41(m, 1H), 5.04(sept, J=6.3Hz, 1H).
   IR(neat):
      3401, 2980, 2928, 2854, 2236, 1727, 1451, 1412, 1375, 1280, 1145, 1106, 1013, 966, 894, 693cm⁻¹.

### Example 3

### Preparation of 3-thia-9-deoxy-9β-chloro-16,17,18,19,20-pentanor-15-cyclohexyl-13,14-didehydro-PGF₁α n-butyl ester

(1) Following a manner similar to that of Example 1(2) using the compound obtained in Example 1(1) and 4-thia-5-carbo-n-butoxypentyl zinc (II) iodide, 3-thia-16,17,18,19,20-pentanor-15-cyclohexyl-13,14-didehydro-PGE₁ n-butyl ester 11,15-bis(t-butyldimethylsilyl ether) was obtained.
   ¹H-NMR(CDCl₃, 200MHz) δ ppm; 0.08(s, 3H), 0.09(s, 3H), 0.10(s, 3H), 0.12(s, 3H), 0.75-1.95(m, 24H), 0.89(s, 9H), 0.90(s, 9H), 2.08-2.27(m, 2H), 2.53-2.78(m, 4H), 3.20(s, 2H), 4.01-4.20(m, 1H), 4.13(t, J=6.6Hz, 2H), 4.29(q, J=6.7Hz, 1H).
   IR(neat);
      2930, 2856, 2235, 1748, 1472, 1463, 1408, 1385, 1362, 1278, 1257, 1103, 1007, 961, 940, 898, 838, 778, 670cm⁻¹.
(2) Following a manner similar to that of Example 1(3) using the compound obtained in (1), 3-thia-16,17,18,19,20-pentanor-15-cyclohexyl-13,14-didehydro-PGF₁α n-butyl ester 11,15-bis(t-butyldimethylsilyl ether) and 3-thia-16,17,18,19,20-pentanor-15-cyclohexyl-13,14-didehydro-PGF₁β n-butyl ester 11,15-bis(t-butyldimethylsilyl ether) were obtained.
   3-Thia-16,17,18,19,20-pentanor-15-cyclohexyl-13,14-didehydro-PGF₁α n-butyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.11(s, 3H), 0.75-2.12(m, 27H), 0.88(s, 9H), 0.90(s, 9H), 2.40-2.50(m, 1H), 2.65(t, J=7.1Hz, 2H), 3.21(s, 2H), 4.03-4.20(m, 1H), 4.07(dd, J=6.4, 1.9Hz, 1H), 4.13(t, J=6.6Hz, 2H), 4.24-4.32(m, 1H).
   IR(neat):
      3469, 2930, 2856, 2231, 1733, 1472, 1463, 1387, 1361, 1278, 1256, 1102, 1069, 1006, 964, 939, 899, 838, 778, 669cm⁻¹.

   3-Thia-16,17,18,19,20-pentanor-15-cyclohexyl-13,14-didehydro-PGF₁β n-butyl ester 11,15-bis(t-butyldimethylsilyl ether)
   ¹H-NMR(CDCl₃, 200MHz) δ ppm; 0.07(s, 3H), 0.08(s, 6H), 0.11(s, 3H), 0.75-1.96(m, 27H), 0.88(s, 9H), 0.90(s, 9H), 2.22(ddd, J=9.4, 6.2, 1.7Hz, 1H), 2.64(t, J=6.9Hz, 2H), 3.21(s, 2H), 3.91-4.04(m, 1H), 4.07(dd, J=6.2, 1.8Hz, 1H), 4.13(t, J=6.6Hz, 2H), 4.15-4.28(m, 1H).
   IR(neat):
      3436, 2955, 2929, 2856, 2232, 1733, 1472, 1463, 1386, 1361, 1280, 1255, 1104, 1066, 1007, 898, 837, 778, 670cm⁻¹.
(3) Following a manner similar to that of Example 1(4) using 3-thia-16,17,18,19,20-pentanor-15-cyclohexyl-13,14-didehydro-PGF₁α n-butyl ester 11,15-bis(t-butyldimethylsilyl ether) obtained in (2), 3-thia-9-deoxy-9β-chloro-16,17,18,19,20-pentanor-15-cyclohexyl-13,14-didehydro-PGF₁α n-butyl ester 11,15-bis(t-butyldimethylsilyl ether) was obtained.
   ¹H-NMR(CDCl₃, 200MHz) δ ppm; 0.07(s, 3H), 0.08(s, 6H), 0.11(s, 3H), 0.76-1.90(m, 24H), 0.88(s, 9H), 0.90(s, 9H), 1.98-2.19(m, 3H), 2.28(ddd, J=8.9, 5.0, 1.6Hz, 1H), 2.64(t, J=7.0Hz, 2H), 3.21(s, 2H), 3.88-4.02(m, 1H), 4.07(dd, J=6.2, 1.6Hz, 1H), 4.14(t, J=6.7Hz, 2H), 4.18-4.29(m, 1H).
   IR(neat):
      2955, 2930, 2856, 2233, 1734, 1472, 1463, 1386, 1362, 1278, 1256, 1101, 1072, 1006, 962, 898, 837, 778, 669cm⁻¹.
(4) Following a manner similar to that of Example 1(5) using the compound obtained in (3), 3-thia-9-deoxy-9β-chloro-16,17,18,19,20-pentanor-15-cyclohexyl-13,14-didehydro-PGF₁α n-butyl ester was obtained.
   ¹H-NMR(CDCl₃, 300MHz) δ ppm; 0.87-1.89(m, 24H), 1.92-2.38(m, 4H), 2.66(t, J=6.9Hz, 2H), 3.22(s, 2H), 3.89-3.99(m, 1H), 4.08-4.23(m, 1H), 4.14(t, J=6.7Hz, 2H), 4.31-4.41(m, 1H).
   IR(neat):
      3401, 2929, 2854, 2235, 1730, 1451, 1413, 1384, 1281, 1133, 1084, 1013, 894, 694cm⁻¹.

### Example 4

### Preparation of 3-thia-9-deoxy-9β-chloro-16,17,18,19,20-pentanor-15-cyclohexyl-13,14-didehydro-PGF₁α t-butyl ester

(1) Following a manner similar to that of Example 1(2) using the compound obtained in Example 1(1) and 4-thia-5-carbo-t-butoxypentyl zinc (II) iodide, 3-thia-16,17,18,19,20-pentanor-15-cyclohexyl-13,14-didehydro-PGE₁ t-butyl ester 11,15-bis(t-butyldimethylsilyl ether) was obtained.
   ¹H-NMR(CDCl₃, 200MHz) δ ppm; 0.08(s, 3H), 0.09(s, 3H), 0.10(s, 3H), 0.12(s, 3H), 0.75-1.95(m, 17H), 0.89(s, 9H), 0.90(s, 9H), 1.47(s, 9H), 1.99-2.27(m, 2H), 2.48-2.85(m, 4H), 3.11(s, 2H), 4.08(dd, J=6.3, 1.5Hz, 1H), 4.29(q, J=6.8Hz, 1H).
   IR(neat):
      2930, 2856, 1728, 1462, 1410, 1392, 1369, 1293, 1256, 1213, 1139, 951, 898, 838, 778, 671cm⁻¹.
(2) Following a manner similar to that of Example 1(3) using the compound obtained in (1), 3-thia-16,17,18,19,20-pentanor-15-cyclohexyl-13,14-didehydro- PGF₁α t-butyl ester 11,15-bis(t-butyldimethylsilyl ether) was 3-thia-16,17,18,19,20-pentanor-15-cyclohexyl-13,14-didehydro-PGF₁β t-butyl ester 11,15-bis(t-butyldimethylsilyl ether) were obtained.
   3-Thia-16,17,18,19,20-pentanor-15-cyclohexyl-13,14-didehydro-PGF₁α t-butyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.11(s, 3H), 0.75-2.10(m, 20H), 0.88(s, 9H), 0.90(s, 9H), 1.47(s, 9H), 2.40-2.50(m, 1H), 2.65(t, J=7.1Hz, 2H), 3.12(s, 2H), 4.04-4.16(m, 1H), 4.07(dd, J=6.3, 1.9Hz, 1H), 4.22-4.32(m, 1H).
   IR(neat):
      3468, 2929, 2856, 2232, 1727, 1472, 1463, 1391, 1369, 1294, 1256, 1127, 1103, 1071, 1006, 953, 898, 838, 778, 669cm⁻¹.

   3-Thia-16,17,18,19,20-pentanor-15-cyclohexyl-13,14-didehydro-PGF₁β t-butyl ester 11,15-bis(t-butyldimethylsilyl ether)
   ¹H-NMR(CDCl₃, 200MHz) δ ppm; 0.07(s, 3H), 0.08(s, 6H), 0.11(s, 3H), 0.75-1.95(m, 20H), 0.88(s, 9H), 0.90(s, 9H), 1.47(s, 9H), 2.23(ddd, J=9.3, 6.3, 1.7Hz, 1H), 2.64(t, J=6.9Hz, 2H), 3.12(s, 2H), 3.98(q, J=6.3Hz, 1H), 4.08(dd, J=6.3, 1.7Hz, 1H), 4.22(q, J=6.3Hz, 1H).
   IR(neat):
      3436, 2929, 2856, 2233, 1726, 1473, 1463, 1391, 1369, 1294, 1255, 1127, 1070, 1006, 898, 837, 778, 669cm⁻¹.
(3) Following a manner similar to that of Example 1(4) using 3-thia-16,17,18,19,20-pentanor-15-cyclohexyl-13,14-didehydro-PGF₁α t-butyl ester 11,15-bis(t-butyldimethylsilyl ether) obtained in (2), 3-thia-9-deoxy-9β-chloro-16,17,18,19,20-pentanor-15-cyclohexyl-13,14-didehydro-PGF₁α t-butyl ester 11,15-bis(t-butyldimethylsilyl ether) was obtained.
   ¹H-NMR(CDCl₃, 200MHz) δ ppm; 0.07(s, 3H), 0.08(s, 6H), 0.11(s, 3H), 0.73-2.20(m, 20H), 0.88(s, 9H), 0.90(s, 9H), 1.48(s, 9H), 2.28(ddd, J=8.9, 4.8, 1.7Hz, 1H), 2.55-2.72(m, 2H), 3.12(s, 2H), 3.95(q, J=7.7Hz, 1H), 4.07(dd, J=6.2, 1.7Hz, 1H), 4.18-4.30(m, 1H).
   IR(neat):
      2930, 2856, 2233, 1728, 1472, 1463, 1391, 1369, 1292, 1255, 1126, 1102, 1073, 1006, 952, 899, 838, 778, 669cm⁻¹.
(4) Following a manner similar to that of Example 1(5) using the compound obtained in (3), 3-thia-9-deoxy-9β-chloro-16,17,18,19,20-pentanor-15-cyclohexyl-13,14-didehydro-PGF₁α t-butyl ester was obtained.
   ¹H-NMR(CDCl₃, 300MHz) δ ppm; 0.96-1.93(m, 17H), 1.48(s, 9H), 1.88(s, 2H), 2.08-2.36(m, 4H), 2.65(t, J=6.9Hz, 2H), 3.13(s, 2H), 3.95(dd, J=14.4, 7.5Hz, 1H), 4.16(dd, J=6.0, 1.9Hz, 1H), 4.37(q, J=6.5Hz, 1H).
   IR(neat):
      3401, 2978, 2928, 2854, 2235, 1724, 1452, 1393, 1369, 1297, 1259, 1137, 1085, 1012, 952, 894, 834, 765cm⁻¹.

### Example 5

### Preparation of 3-thia-9-deoxy-9β-chloro-16,17,18,19,20-pentanor-15-cyclohexyl-13,14-didehydro-PGF₁α cyclohexyl ester

(1) Following a manner similar to that of Example 1(2) using the compound obtained in Example 1(1) and 4-thia-5-carbocyclohexyloxypentyl zinc (II) iodide, 3-thia-16,17,18,19,20-pentanor-15-cyclohexyl-13,14-didehydro-PGE₁ cyclohexyl ester 11,15-bis(t-butyldimethylsilyl ether) was obtained.
   ¹H-NMR(CDCl₃, 200MHz) δ ppm; 0.08(s, 3H), 0.09(s, 3H), 0.10(s, 3H), 0.12(s, 3H), 0.76-2.28(m, 28H), 0.89(s, 9H), 0.90(s, 9H), 2.17(dd, J=18.7, 7.0Hz, 1H), 2.57-2.83(m, 4H), 3.20(s, 2H), 4.04-4.18(m, 1H), 4.22-4.35(m, 1H), 4.72-4.94(m, 1H).
   IR(neat):
      2931, 2857, 2236, 1747, 1463, 1451, 1409, 1386, 1280, 1259, 1215, 1122, 1040, 1016, 973, 928, 897, 838, 779, 670cm⁻¹.
(2) Following a manner similar to that of Example 1(3) using the compound obtained in (1), 3-thia-16,17,18,19,20-pentanor-15-cyclohexyl-13,14-didehydro-PGF₁α cyclohexyl ester 11,15-bis(t-butyldimethylsilyl ether) and 3-thia-16,17,18,19,20-pentanor-15-cyclohexyl-13,14-didehydro-PGF₁β cyclohexyl ester 11,15-bis(t-butyldimethylsilyl ether) were obtained.
   3-Thia-16,17,18,19,20-pentanor-15-cyclohexyl-13,14-didehydro-PGF₁α cyclohexyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.11(s, 3H), 0.55-2.08(m, 30H), 0.88(s, 9H), 0.90(s, 9H), 2.11(br s, 1H), 2.40-2.50(m, 1H), 2.66(t, J=7.0Hz, 2H), 3.19(s, 2H), 4.02-4.20(m, 1H), 4.07(dd, J=6.3, 2.0Hz, 1H), 4.22-4.32(m, 1H), 4.70-4.94(m, 1H).
   IR(neat):
      3524, 2930, 2856, 2232, 1728, 1472, 1451, 1361, 1257, 1102, 1071, 1015, 965, 898, 838, 778, 669cm⁻¹.

   3-Thia-16,17,18,19,20-pentanor-15-cyclohexyl-13,14-didehydro-PGF₁β cyclohexyl ester 11,15-bis(t-butyldimethylsilyl ether)
   ¹H-NMR(CDCl₃, 200MHz) δ ppm; 0.07(s, 3H), 0.08(s, 6H), 0.11(s, 3H), 0.83-2.10(m, 30H), 0.88(s, 9H), 0.90(s, 9H), 2.23(ddd, J=9.3, 6.2, 1.6Hz, 1H), 2.65(t, J=6.9Hz, 2H), 3.19(s, 2H), 3.90-4.05(m, 1H), 4.08(dd, J=6.2, 1.6Hz, 1H), 4.15-4.32(m, 1H), 4.70-4.91(m, 1H).
   IR(neat):
      3456, 2930, 2856, 2233, 1727, 1472, 1463, 1451, 1361, 1282, 1256, 1122, 1069, 1015, 963, 898, 837, 778, 669cm⁻¹.
(3) Following a manner similar to that of Example 1(4) using 3-thia-16,17,18,19,20-pentanor-15-cyclohexyl-13,14-didehydro-PGF₁α t-butyl ester 11,15-bis(t-butyldimethylsilyl ether) obtained in (2), 3-thia-9-deoxy-9β-chloro-16,17,18,19,20-pentanor-15-cyclohexyl-13,14-didehydro-PGF₁α cyclohexyl ester 11,15-bis(t-butyldimethylsilyl ether) was obtained.
   ¹H-NMR(CDCl₃, 200MHz) δ ppm; 0.07(s, 3H), 0.08(s, 3H), 0.10(s, 3H), 0.11(s, 3H), 0.76-2.19(m, 30H), 0.88(s, 9H), 0.90(s, 9H), 2.28(ddd, J=9.0, 4.8, 1.5Hz, 1H), 2.65(t, J=7.0Hz, 2H), 3.19(s, 2H), 3.87-4.03(m, 1H), 4.07(dd, J=6.2, 1.6Hz, 1H), 4.18-4.31(m, 1H), 4.70-4.91(m, 1H).
   IR(neat):
      2931, 2857, 2234, 1729, 1472, 1463, 1451, 1409, 1386, 1361, 1338, 1279, 1258, 1100, 1073, 1017, 963, 898, 837, 778, 670cm⁻¹.
(4) Following a manner similar to that of Example 1(5) using the compound obtained in (3), 3-thia-9-deoxy-9β-chloro-16,17,18,19,20-pentanor-15-cyclohexyl-13,14-didehydro-PGF₁α cyclohexyl ester was obtained.
   ¹H-NMR(CDCl₃, 300MHz) δ ppm; 0.94-1.94(m, 26H), 2.01-2.35(m, 2H), 2.19(ddd, J=13.9, 7.8, 6.1Hz, 1H), 2.27(dt, J=13.9, 6.9Hz, 1H), 2.30(ddd, J=9.9, 6.5, 2.0Hz, 1H), 2.66(t, J=7.0Hz, 2H), 3.19(s, 2H), 3.89-3.96(m, 1H), 4.10-4.20(m, 1H), 4.31-4.44(m, 1H), 4.72-4.84(m, 1H).
   IR(neat):
      3401, 2931, 2856, 2236, 1724, 1451, 1412, 1284, 1123, 1085, 1038, 1015, 974, 893, 757cm⁻¹.

### Example 6

### Preparations of 3-thia-9-deoxy-9β-chloro-16,17,18,19,20-pentanor-15-cyclopentyl-13,14-didehydro-PGF₁α and methyl ester thereof

(1) Following a manner similar to that of Example 1(2) using (3R,4R)-2-methylene-3-[(3'S)-3'-(t-butyldimethylsiloxy)-3'-cyclopentylprop-1'-ynyl]-4-(t-butyldimethylsiloxy)cyclopentan-1-one, 3-thia-16,17,18,19,20-pentanor-15-cyclopentyl-13,14-didehydro-PGE₁ methyl ester 11,15-bis(t-butyldimethylsilyl ether) was obtained.
   ¹H-NMR(CDCl₃, 200MHz) δ ppm; 0.09(s, 6H), 0.11(s, 3H), 0.13(s, 3H), 1.15-1.85(m, 14H), 0.89(s, 9H), 0.90(s, 9H), 2.02-2.25(m, 2H), 2.17(dd, J=18.3, 6.9Hz, 1H), 2.58-2.79(m, 4H), 3.22(s, 2H), 3.74(s, 3H), 4.18(dd, J=6.8, 1.4Hz, 1H), 4.22-4.35(m, 1H).
   IR(neat):
      2954, 2931, 2858, 2234, 1747, 1472, 1463, 1437, 1408, 1362, 1280, 1256, 1131, 1100, 1007, 940, 838, 778, 671cm⁻¹.
(2) Following a manner similar to that of Example 1(3) using the compound obtained in (1), 3-thia-16,17,18,19,20-pentanor-15-cyclopentyl-13,14-didehydro-PGF₁α methyl ester 11,15-bis(t-butyldimethylsilyl ether) and 3-thia-16,17,18,19,20-pentanor-15-cyclopentyl-13,14-didehydro-PGF₁β methyl ester 11,15-bis(t-butyldimethylsilyl ether) were obtained.
   3-Thia-16,17,18,19,20-pentanor-15-cyclopentyl-13,14-didehydro-PGF₁α methyl ester 11,15-bis(t-butyldimethylsilyl ether)
   ¹H-NMR(CDCl₃, 200MHz) δ ppm; 0.09(s, 3H), 0.10(s, 3H), 0.11(s, 6H), 0.89(s, 9H), 0.90(s, 9H), 1.18-2.19(m, 18H), 2.39-2.49(m, 1H), 2.66(t, J=7.0Hz, 2H), 3.23(s, 2H), 3.74(s, 3H), 4.07-4.19(m, 1H), 4.17(dd, J=6.9, 2.0Hz, 1H), 4.23-4.31(m, 1H).
   IR(neat):
      3523, 2954, 2931, 2858, 2230, 1740, 1472, 1463, 1437, 1387, 1361, 1278, 1256, 1100, 1071, 1006, 940, 838, 778, 670cm⁻¹.

   3-Thia-16,17,18,19,20-pentanor-15-cyclopentyl-13,14-didehydro-PGF₁β methyl ester 11,15-bis(t-butyldimethylsilyl ether)
   ¹H-NMR(CDCl₃, 200MHz) δ ppm; 0.07(s, 3H), 0.09(s, 6H), 0.12(s, 3H), 0.88(s, 9H), 0.90(s, 9H), 1.23-1.93(m, 17H), 2.01-2.24(m, 1H), 2.22(ddd, J=9.2, 6.1, 1.6Hz, 1H), 2.65(t, J=6.9Hz, 2H), 3.23(s, 2H), 3.74(s, 3H), 3.92-4.03(m, 1H), 4.13-4.28(m, 2H).
   IR(neat):
      3458, 2954, 2930, 2857, 2231, 1740, 1473, 1463, 1438, 1388, 1361, 1281, 1255, 1131, 1071, 1007, 940, 838, 778, 671cm⁻¹.
(3) Following a manner similar to that of Example 1(4) using 3-thia-16,17,18,19,20-pentanor-15-cyclopentyl-13,14-didehydro-PGF₁α methyl ester 11,15-bis(t-butyldimethylsilyl ether) obtained in (2), 3-thia-9-deoxy-9β-chloro-16,17,18,19,20-pentanor-15-cyclopentyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 6H), 0.12(s, 3H), 0.88(s, 9H), 0.90(s, 9H), 1.22-1.80(m, 14H), 1.97-2.18(m, 4H), 2.27(ddd, J=8.8, 4.7, 1.6Hz, 1H), 2.64(t, J=6.9Hz, 2H), 3.23(s, 2H), 3.74(s, 3H), 3.88-4.02(m, 1H), 4.16-4.28(m, 1H), 4.17(dd, J=6.8, 1.6Hz, 1H).
   IR(neat):
      2954, 2931, 2858, 2231, 1741, 1472, 1463, 1437, 1408, 1387, 1362, 1279, 1256, 1131, 1073, 1007, 940, 909, 838, 778, 671cm⁻¹.
(4) Following a manner similar to that of Example 1(5) using the compound obtained in (3), 3-thia-9-deoxy-9β-chloro-16,17,18,19,20-pentanor-15-cyclopentyl-13,14- didehydro-PGF₁α methyl ester was obtained.
   ¹H-NMR(CDCl₃, 300MHz) δ ppm; 1.36-1.84(m, 14H), 2.06-2.33(m, 4H), 2.30(ddd, J=9.8, 6.4, 1.9Hz, 1H), 2.66(t, J=7.0Hz, 2H), 3.23(s, 2H), 3.75(s, 3H), 3.90-3.99(m, 1H), 4.25(dd, J=6.9, 1.5Hz, 1H), 4.31-4.41(m, 1H).
   IR(neat):
      3401, 2951, 2864, 2234, 1734, 1438, 1385, 1283, 1137, 1025, 757cm⁻¹.
(5) Following a manner similar to that of Example 1(6) using the compound obtained in (4), 3-thia-9-deoxy-9β-chloro-16,17,18,19,20-pentanor-15-cyclopentyl-13,14-didehydro-PGF₁α was obtained.
   ¹H-NMR(CDCl₃, 300MHz) δ ppm; 1.29-1.86(m, 14H), 2.08-2.37(m, 4H), 2.32(ddd, J=10.0, 6.5, 1.7Hz, 1H), 2.54-2.90(m, 2H), 3.25(br s, 2H), 3.90-4.00(m, 1H), 4.28(dd, J=7.1, 1.7Hz, 1H), 4.28-4.41(m, 1H).
   IR(KBr):
      3369, 2946, 2854, 2221, 1712, 1679, 1459, 1416, 1388, 1356, 1307, 1247, 1215, 1194, 1172, 1140, 1091, 1030, 957, 870, 800, 726, 677, 582cm⁻¹.

### Example 7

### Preparations of 3-thia-9-deoxy-9β-chloro-16,17,18,19,20-pentanor-15-cycloheptyl-13,14-didehydro-PGF₁α and methyl ester thereof

(1) Following a manner similar to that of Example 1(2) using (3R,4R)-2-methylene-3-[(3'S)-3'-(t-butyldimethylsiloxy)-3'-cycloheptylprop-1'-ynyl]-4-(t-butyldimethylsiloxy)cyclopentan-1-one, 3-thia-16,17,18,19,20-pentanor-15-cycloheptyl-13,14-didehydro-PGE₁ methyl ester 11,15-bis(t-butyldimethylsilyl ether) was obtained.
   ¹H-NMR(CDCl₃, 200MHz) δ ppm; 0.08(s, 3H), 0.09(s, 3H), 0.10(s, 3H), 0.13(s, 3H), 0.89(s, 18H), 1.15-1.96(m, 19H), 2.01-2.28(m, 1H), 2.17(dd, J=18.2, 6.8Hz, 1H), 2.46-2.80(m, 4H), 3.22(s, 2H), 3.74(s, 3H), 4.17(dd, J=5.3, 1.5Hz, 1H), 4.20-4.36(m, 1H).
   IR(neat):
      2953, 2929, 2857, 2232, 1747, 1472, 1463, 1437, 1408, 1385, 1362, 1280, 1255, 1130, 1086, 1007, 940, 838, 778, 670cm⁻¹.
(2) Following a manner similar to that of Example 1(3) using the compound obtained in (1), 3-thia-16,17,18,19,20-pentanor-15-cycloheptyl-13,14-didehydro-PGF₁α methyl ester 11,15-bis(t-butyldimethylsilyl ether) and 3-thia-16,17,18,19,20-pentanor-15-cycloheptyl-13,14-didehydro-PGF₁β methyl ester 11,15-bis(t-butyldimethylsilyl ether) were obtained.
   3-Thia-16,17,18,19,20-pentanor-15-cycloheptyl-13,14-didehydro-PGF₁α methyl ester 11,15-bis(t-butyldimethylsilyl ether)
   ¹H-NMR(CDCl₃, 200MHz) δ ppm; 0.08(s, 3H), 0.09(s, 6H), 0.11(s, 3H), 0.88(s, 9H), 0.89(s, 9H), 1.15-2.07(m, 22H), 2.38-2.49(m, 1H), 2.66(t, J=7.1Hz, 2H), 3.23(s, 2H), 3.74(s, 3H), 4.07-4.20(m, 1H), 4.14(dd, J=5.5, 2.0Hz, 1H), 4.23-4.33(m, 1H).
   IR(neat):
      3524, 2929, 2857, 2229, 1740, 1472, 1463, 1437, 1388, 1361, 1279, 1255, 1131, 1084, 1006, 939, 838, 778, 669cm⁻¹.

   3-Thia-16,17,18,19,20-pentanor-15-cycloheptyl-13,14-didehydro-PGF₁β methyl ester 11,15-bis(t-butyldimethylsilyl ether)
   ¹H-NMR(CDCl₃, 200MHz) δ ppm; 0.07(s, 6H), 0.09(s, 3H), 0.11(s, 3H), 0.88(s, 9H), 0.89(s, 9H), 1.14-1.95(m, 22H), 2.22(ddd, J=9.2, 6.1, 1.6Hz, 1H), 2.64(t, J=6.9Hz, 2H), 3.23(s, 2H), 3.74(s, 3H), 3.90-4.04(m, 1H), 4.12-4.30(m, 1H), 4.16(dd, J=5.4, 1.6Hz, 1H).
   IR(neat):
      3459, 2928, 2857, 2231, 1740, 1472, 1463, 1438, 1388, 1361, 1281, 1254, 1131, 1082, 1007, 838, 777, 670cm⁻¹.
(3) Following a manner similar to that of Example 1(4) using 3-thia-16,17,18,19,20-pentanor-15-cycloheptyl-13,14-didehydro-PGF₁α methyl ester 11,15-bis(t-butyldimethylsilyl ether) obtained in (2), 3-thia-9-deoxy-9β-chloro-16,17,18,19,20-pentanor-15-cycloheptyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.09(s, 3H), 0.11(s, 3H), 0.88(s, 9H), 0.90(s, 9H), 1.14-1.90(m, 19H), 1.95-2.20(m, 3H), 2.28(ddd, J=8.8, 4.6, 1.7Hz, 1H), 2.63(t, J=7.3Hz, 2H), 3.23(s, 2H), 3.74(s, 3H), 3.87-4.03(m, 1H), 4.15(dd, J=5.4, 1.7Hz, 1H), 4.17-4.28(m, 1H).
   IR(neat):
      2929, 2857, 2231, 1741, 1472, 1463, 1437, 1387, 1362, 1279, 1256, 1130, 1084, 1007, 940, 837, 778, 671cm⁻¹.
(4) Following a manner similar to that of Example 1(5) using the compound obtained in (3), 3-thia-9-deoxy-9β-chloro-16,17,18,19,20-pentanor-15-cycloheptyl-13,14-didehydro-PGF₁α methyl ester was obtained.
   ¹H-NMR(CDCl₃, 300MHz) δ ppm; 1.22-1.91(m, 19H), 2.07-2.34(m, 1H), 2.19(ddd, J=14.0, 7.8, 6.1Hz, 1H), 2.27(dt, J=14.0, 6.9Hz, 1H), 2.30(ddd, J=9.8, 6.3, 1.8Hz, 1H), 2.66(t, J=7.0Hz, 2H), 3.23(s, 2H), 3.75(s, 3H), 3.90-4.00(m, 1H), 4.23(dd, J=5.4, 1.8Hz, 1H), 4.32-4.40(m, 1H).
   IR(neat):
      3401, 2926, 2856, 2233, 1734, 1438, 1385, 1282, 1196, 1135, 1048, 1014, 687, 594cm⁻¹.
(5) Following a manner similar to that of Example 1(6) using the compound obtained in (4), 3-thia-9-deoxy-9β-chloro-16,17,18,19,20-pentanor-15-cycloheptyl-13,14-didehydro-PGF₁α was obtained.
   ¹H-NMR(CDCl₃, 300MHz) δ ppm; 1.20-1.90(m, 19H), 2.09-2.36(m, 1H), 2.20(ddd, J=14.0, 7.8, 6.0Hz, 1H), 2.27(dt, J=14.0, 6.8Hz, 1H), 2.32(ddd, J=9.8, 6.4, 1.9Hz, 1H), 2.63-2.77(m, 2H), 3.24(s, 2H), 3.90-4.00(m, 1H), 4.27(dd, J=5.4, 1.8Hz, 1H), 4.31-4.40(m, 1H).
   IR(neat):
      3369, 2921, 2852, 2234, 1711, 1462, 1385, 1280, 1048cm⁻¹.

### Example 8

### Preparations of (2'RS)-3-thia-9-deoxy-9β-chloro-16,17,18,19,20-pentanor-15-(2'-methylcyclohexyl)-13,14-didehydro-PGF₁α and methyl ester thereof

(1) Following a manner similar to that of Example 1(2) using (3R,4R)-2-methylene-3-[(3'S)-3'-(t-butyldimethylsiloxy)-3'-((2''RS)-2''-methylcycloheptylprop)-1'-ynyl]-4-(t-butyldimethylsiloxy)cyclopentan-1-one, (2'RS)-3-thia-16,17,18,19,20-pentanor-15-(2'-methylcyclohexyl)-13,14-didehydro-PGE₁ methyl ester 11,15-bis(t-butyldimethylsilyl ether) was obtained.
   ¹H-NMR(CDCl₃, 200MHz) δ ppm; 0.09, 0.10, 0.11, 0.13 and 0.14(5s, 12H), 0.85 and 0.86(d, J=7.1Hz, 3H), 0.89(s, 9H), 0.91(s, 9H), 0.95-1.84(m, 15H), 2.08-2.28(m, 2H), 2.17(dd, J=18.0, 7.1Hz, 1H), 2.57-2.76(m, 3H), 2.68(dd, J=18.0, 6.7Hz, 1H), 3.22(s, 2H), 3.74(s, 3H), 3.95-4.12(m, 1H), 4.22-4.35(m, 1H).
   IR(neat):
      2953, 2929, 2857, 2233, 1747, 1472, 1463, 1437, 1408, 1383, 1362, 1279, 1256, 1131, 1103, 1063, 1006, 939, 838, 778, 670cm⁻¹.
(2) Following a manner similar to that of Example 1(3) using the compound obtained in (1), (2'RS)-3-thia-16,17,18,19,20-pentanor-15-(2'-methylcyclohexyl)-13,14-didehydro-PGF₁α methyl ester 11,15-bis(t-butyldimethylsilyl ether) and (2'RS)-3-thia-16,17,18,19,20-pentanor-15-(2'-methylcyclohexyl)-13,14-didehydro-PGF₁β methyl ester 11,15-bis(t-butyldimethylsilyl ether) were obtained.
   (2'RS)-3-Thia-16,17,18,19,20-pentanor-15-(2'-methylcyclohexyl)-13,14-didehydro-PGF₁α methyl ester 11,15-bis(t-butyldimethylsilyl ether)
   ¹H-NMR(CDCl₃, 200MHz) δ ppm; 0.08, 0.09, 0.10 and 0.13(4s, 12H), 0.75-0.94(m, 3H), 0.88, 0.89 and 0.90(3s, 18H), 0.98-2.27(m, 19H), 2.40-2.49(m, 1H), 2.65(t, J=7.0Hz, 2H), 3.23(s, 2H), 3.74(s, 3H), 4.01-4.15(m, 2H), 4.24-4.31(m, 1H).
   IR(neat):
      3523, 2928, 2857, 2229, 1740, 1472, 1463, 1437, 1387, 1362, 1256, 1102, 1062, 1006, 939, 838, 778, 669cm⁻¹.

   (2'RS)-3-Thia-16,17,18,19,20-pentanor-15-(2'-methylcyclohexyl)-13,14-didehydro-PGF₁β methyl ester 11,15-bis(t-butyldimethylsilyl ether)
   ¹H-NMR(CDCl₃, 200MHz) δ ppm; 0.07, 0.08, 0.09, 0.10, 0.12 and 0.15(6s, 12H), 0.76-1.94(m, 21H), 0.88, 0.89 and 0.90(3s, 18H), 2.13-2.29(m, 2H), 2.64(t, J=6.9Hz, 2H), 3.23(s, 2H), 3.74(s, 3H), 3.92-4.09(m, 2H), 4.16-4.26(m, 1H).
   IR(neat):
      3467, 2954, 2928, 2857, 2230, 1740, 1472, 1463, 1438, 1385, 1361, 1281, 1255, 1132, 1103, 1063, 1006, 838, 778, 669cm⁻¹.
(3) Following a manner similar to that of Example 1(4) using (2'RS)-3-thia-16,17,18,19,20-pentanor-15-(2'-methylcyclohexyl)-13,14-didehydro-PGF₁α methyl ester 11,15-bis(t-butyldimethylsilyl ether) obtained in (2), (2'RS)-3-thia-9-deoxy-9β-chloro-16,17,18,19,20-pentanor-15-(2'-methylcyclohexyl)-13,14-didehydro-PGF₁α methyl ester 11,15-bis(t-butyldimethylsilyl ether) was obtained.
   ¹H-NMR(CDCl₃, 200MHz) δ ppm; 0.09, 0.10, 0.12, 0.14 and 0.16(5s, 12H), 0.68-0.93(m, 3H), 0.89, 0.91 and 0.92(3s, 18H), 1.00-2.35(m, 20H), 2.60-2.72(m, 2H), 3.24(s, 2H), 3.76(s, 3H), 3.89-4.11(m, 2H), 4.20-4.31(m, 1H).
   IR(neat):
      2953, 2929, 2857, 2232, 1741, 1472, 1463, 1437, 1385, 1362, 1279, 1256, 1132, 1101, 1063, 1006, 939, 907, 838, 778, 669cm⁻¹.
(4) Following a manner similar to that of Example 1(5) using the compound obtained in (3), (2'RS)-3-thia-9-deoxy-9β-chloro-16,17,18,19,20-pentanor-15-(2'-methylcyclohexyl)-13,14-didehydro-PGF₁α methyl ester was obtained.
   ¹H-NMR(CDCl₃, 300MHz) δ ppm; 0.88 and 0.91(2d, J=7.3Hz, 3H), 1.15-1.88(m, 15H), 2.06-2.35(m, 4H), 2.31(ddd, J=9.7, 6.4, 2.0Hz, 1H), 2.66(t, J=6.7Hz, 2H), 3.24(s, 2H), 3.75(s, 3H), 3.90-4.00(m, 1H), 4.03-4.15(m, 1H), 4.32-4.41(m, 1H).
   IR(neat):
      3401, 2926, 2857, 2233, 1738, 1439, 1384, 1283, 1199, 1136, 1087, 1017, 979, 694, 594cm⁻¹.
(5) Following a manner similar to that of Example 1(6) using the compound obtained in (4), (2'RS)-3-thia-9-deoxy-9β-chloro-16,17,18,19,20-pentanor-15-(2'-methylcyclohexyl)-13,14-didehydro-PGF₁α was obtained.
   ¹H-NMR(CDCl₃, 300MHz) δ ppm; 0.88 and 0.91(2d, J=6.8Hz, 3H), 1.10-1.94(m, 15H), 2.07-2.37(m, 5H), 2.58-2.80(m, 2H), 3.24(br s, 2H), 3.90-4.00(m, 1H), 4.06-4.19(m, 1H), 4.31-4.41(m, 1H).
   IR(neat):
      3368, 2926, 2857, 2235, 1713, 1446, 1380, 1280, 1156, 1087, 1019, 977, 681cm⁻¹.

### Example 9

### Preparations of 3-thia-9-deoxy-9β-chloro-17,18,19,20-tetranor-16-cyclopentyl-13,14-didehydro-PGF₁α and methyl ester thereof

(1) Following a manner similar to that of Example 1(2) using (3R,4R)-2-methylene-3-[(3'S)-3'-(t-butyldimethylsiloxy)-4'-cyclopentylbut-1'-ynyl]-4-(t-butyldimethylsiloxy)cyclopentan-1-one, 3-thia-17,18,19,20-tetranor-16-cyclopentyl-13,14-didehydro-PGE₁ methyl ester 11,15-bis(t-butyldimethylsilyl ether) was obtained.
   ¹H-NMR(CDCl₃, 200MHz) δ ppm; 0.10(s, 6H), 0.11(s, 3H), 0.13(s, 3H), 0.89(s, 9H), 0.90(s, 9H), 0.92-2.27(m, 18H), 2.17(dd, J=18.2, 6.9Hz, 1H), 2.57-2.74(m, 4H), 3.22(s, 2H), 3.74(s, 3H), 4.22-4.41(m, 2H).
   IR(neat):
      2952, 2934, 2858, 2234, 1747, 1472, 1463, 1437, 1408, 1362, 1280, 1256, 1131, 1102, 1007, 940, 838, 779, 670cm⁻¹.
(2) Following a manner similar to that of Example 1(3) using the compound obtained in (1), 3-thia-17,18,19,20-tetranor-16-cyclopentyl-13,14-didehydro-PGF₁α ester 11,15-bis(t-butyldimethylsilyl ether) and 3-thia-17,18,19,20-tetranor-16-cyclopentyl-13,14-didehydro-PGF₁β methyl ester 11,15-bis(t-butyldimethylsilyl ether) was obtained.
   3-Thia-17,18,19,20-tetranor-16-cyclopentyl-13,14-didehydro-PGF₁α methyl ester 11,15-bis(t-butyldimethylsilylether)
   ¹H-NMR(CDCl₃, 200MHz) δ ppm; 0.09(s, 3H), 0.10(s, 6H), 0.11(s, 3H), 0.88(s, 9H), 0.90(s, 9H), 1.00-2.02(m, 20H), 2.40-2.49(m, 1H), 2.65(t, J=7.1Hz, 2H), 3.23(s, 2H), 3.74(s, 3H), 4.06-4.15(m, 1H), 4.23-4.31(m, 1H), 4.35(dt, J=1.9, 6.9Hz, 1H).
   IR(neat):
      3469, 2952, 2931, 2858, 2230, 1740, 1472, 1463, 1437, 1409, 1388, 1362, 1337, 1279, 1256, 1132, 1102, 1073, 1006, 939, 870, 838, 778, 668cm⁻¹.

   3-Thia-17,18,19,20-tetranor-16-cyclopentyl-13,14-didehydro-PGF₁β methyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.12(s, 3H), 0.88(s, 9H), 0.90(s, 9H), 0.96-2.04(m, 20H), 2.22(ddd, J=9.2, 6.3, 1.5Hz, 1H), 2.65(t, J=6.9Hz, 2H), 3.23(s, 2H), 3.74(s, 3H), 3.91-4.04(m, 1H), 4.16-4.28(m, 1H), 4.36(dt, J=1.5, 6.9Hz, 1H).
   IR(neat):
      3459, 2952, 2930, 2857, 2232, 1740, 1472, 1463, 1438, 1388, 1362, 1281, 1255, 1132, 1103, 1072, 1007, 939, 837, 778, 670cm⁻¹.
(3) Following a manner similar to that of Example 1(4) using 3-thia-17,18,19,20-tetranor-16-cyclopentyl-13,14-didehydro-PGF₁α methyl ester 11,15-bis(t-butyldimethylsilyl ether) obtained in (2), 3-thia-9-deoxy-9β-chloro-17,18,19,20-tetranor-16-cyclopentyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.09(s, 3H), 0.10(s, 3H), 0.12(s, 3H), 0.88(s, 9H), 0.90(s, 9H), 0.98-2.17(m, 20H), 2.27(ddd, J=9.0, 4.9, 1.7Hz, 1H), 2.65(t, J=6.9Hz, 2H), 3.23(s, 2H), 3.74(s, 3H), 3.87-4.02(m, 1H), 4.19-4.31(m, 1H), 4.35(dt, J=1.7, 6.7Hz, 1H).
   IR(neat):
      2952, 2931, 2858, 2233, 1741, 1472, 1463, 1437, 1388, 1362, 1279, 1255, 1131, 1075, 1007, 940, 906, 837, 812, 778, 670cm⁻¹.
(4) Following a manner similar to that of Example 1(5) using the compound obtained in (3), 3-thia-9-deoxy-9β-chloro-17,18,19,20-tetranor-16-cyclopentyl-13,14-didehydro-PGF₁α methyl ester was obtained.
   ¹H-NMR(CDCl₃, 300MHz) δ ppm; 1.06-1.22(m, 2H), 1.46-2.07(m, 15H), 2.09-2.22(m, 1H), 2.19(ddd, J=14.0, 7.9, 6.1Hz, 1H), 2.27(dt, J=14.0, 6.8Hz, 1H), 2.30(ddd, J=9.8, 6.4, 1.8Hz, 1H), 2.66(t, J=6.8Hz, 2H), 3.24(s, 2H), 3.75(s, 3H), 3.90-4.00(m, 1H), 4.32-4.43(m, 1H), 4.39(dt, J=1.8, 6.9Hz, 1H).
   IR(neat):
      3401, 2946, 2860, 2234, 1736, 1642, 1550, 1438, 1385, 1282, 1197, 1135, 1038cm⁻¹.
(5) Following a manner similar to that of Example 1(6) using the compound obtained in (4), 3-thia-9-deoxy-9β-chloro-17,18,19,20-tetranor-16-cyclopentyl-13,14-didehydro-PGF₁α was obtained.
   ¹H-NMR(CDCl₃, 300MHz) δ ppm; 1.05-1.31(m, 2H), 1.46-2.06(m, 15H), 2.09-2.35(m, 1H), 2.19(ddd, J=14.1, 7.8, 6.3Hz, 1H), 2.27(dt, J=14.1, 6.8Hz, 1H), 2.32(ddd, J=10.0, 6.6, 1.8Hz, 1H), 2.70(t, J=6.7Hz, 2H), 3.24(s, 2H), 3.90-3.99(m, 1H), 4.10(br s, 3H), 4.32-4.42(m, 1H), 4.42(dt, J=1.8, 7.0Hz, 1H).
   IR(neat):
      3391, 2943, 2860, 2236, 1709, 1451, 1284, 1140, 1089, 1039, 680cm⁻¹.

### Example 10

### Preparations of 3-thia-9-deoxy-9β-chloro-17,18,19,20-tetranor-16-cyclohexyl-13,14-didehydro-PGF₁α and methyl ester thereof

(1) Following a manner similar to that of Example 1(2) using (3R,4R)-2-methylene-3-[(3'S)-3'-(t-butyldimethylsiloxy)-4'-cyclohexylbut-1'-ynyl]-4-(t-butyldimethylsiloxy)cyclopentan-1-one, 3-thia-17,18,19,20-tetranor-16-cyclohexyl-13,14-didehydro-PGE₁ methyl ester 11,15-bis(t-butyldimethylsilyl ether) was obtained.
   ¹H-NMR (CDCl₃, 200MHz) δ ppm; 0.09(s, 6H), 0.11(s, 3H), 0.13(s, 3H), 0.74-1.86(m, 19H), 0.89(s, 9H), 0.90(s, 9H), 2.07-2.28(m, 1H), 2.17(dd, J=18.2, 7.0Hz, 1H), 2.54-2.78(m, 4H), 3.22(s, 2H), 3.74(s, 3H), 4.22-4.36(m, 1H), 4.38-4.52(m, 1H).
   IR(neat):
      2928, 2856, 2233, 1747, 1472, 1463, 1449, 1362, 1279, 1256, 1131, 1098, 1075, 1006, 939, 838, 779, 670cm⁻¹.
(2) Following a manner similar to that of Example 1(3) using the compound obtained in (1), 3-thia-17,18,19,20-tetranor-16-cyclohexyl-13,14-didehydro-PGF₁α methyl ester 11,15-bis(t-butyldimethylsilyl ether) and 3-thia-17,18,19,20-tetranor-16-cyclohexyl-13,14-didehydro-PGF₁β methyl ester 11,15-bis(t-butyldimethylsilyl ether) was obtained.
   3-Thia-17,18,19,20-tetranor-16-cyclohexyl-13,14-didehydro-PGF₁α methyl ester 11,15-bis(t-butyldimethylsilyl ether)
   ¹H-NMR(CDCl₃, 200MHz) δ ppm; 0.09(s, 6H), 0.11(s, 6H), 0.74-2.14(m, 22H), 0.88(s, 9H), 0.90(s, 9H), 2.38-2.49(m, 1H), 2.65(t, J=7.0Hz, 2H), 3.23(s, 2H), 3.74(s, 3H), 4.06-4.16(m, 1H), 4.22-4.32(m, 1H), 4.36-4.49(m, 1H).
   IR(neat):
      3479, 2928, 2856, 2230, 1740, 1472, 1463, 1449, 1388, 1361, 1255, 1131, 1097, 1074, 1005, 939, 838, 778, 668cm⁻¹.

   3-Thia-17,18,19,20-tetranor-16-cyclohexyl-13,14-didehydro-PGF₁β methyl ester 11,15-bis(t-butyldimethylsilyl ether)
   ¹H-NMR(CDCl₃, 200MHz) δ ppm; 0.08(s, 3H), 0.09(s, 3H), 0.10(s, 3H), 0.12(s, 3H), 0.72-1.96(m, 22H), 0.88(s, 9H), 0.90(s, 9H), 2.22(ddd, J=9.2, 6.3, 1.7Hz, 1H), 2.65(t, J=7.0Hz, 2H), 3.23(s, 2H), 3.74(s, 3H), 3.91-4.05(m, 1H), 4.15-4.29(m, 1H), 4.43(dt, J=1.6, 7.5Hz, 1H).
   IR(neat):
      3467, 2928, 2856, 2232, 1740, 1472, 1463, 1449, 1388, 1361, 1280, 1255, 1131, 1073, 1006, 939, 838, 778, 670cm⁻¹.
(3) Following a manner similar to that of Example 1(4) using 3-thia-17,18,19,20-tetranor-16-cyclohexyl-13,14-didehydro-PGF₁α methyl ester 11,15-bis(t-butyldimethylsilyl ether) obtained in (2), 3-thia-9-deoxy-9β-chloro-17,18,19,20-tetranor-16-cyclohexyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.09(s, 3H), 0.10(s, 3H), 0.12(s, 3H), 0.74-1.80(m, 19H), 0.88(s, 9H), 0.90(s, 9H), 1.95-2.19(m, 3H), 2.27(ddd, J=8.8, 4.8, 1.8Hz, 1H), 2.57-2.71(m, 2H), 3.23(s, 2H), 3.74(s, 3H), 3.87-4.04(m, 1H), 4.17-4.31(m, 1H), 4.37-4.49(m, 1H).
   IR(neat):
      2928, 2856, 2232, 1741, 1472, 1463, 1448, 1388, 1361, 1278, 1255, 1130, 1096, 1074, 1006, 939, 891, 837, 811, 778, 670cm⁻¹.
(4) Following a manner similar to that of Example 1(5) using the compound obtained in (3), 3-thia-9-deoxy-9β-chloro-17,18,19,20-tetranor-16-cyclohexyl-13,14-didehydro-PGF₁α methyl ester was obtained.
   ¹H-NMR(CDCl₃, 300MHz) δ ppm; 0.86-1.03(m, 2H), 1.11-1.39(m, 4H), 1.44-1.83(m, 13H), 2.08-2.20(m, 1H), 2.19(ddd, J=14.0, 7.8, 6.1Hz, 1H), 2.27(dt, J=14.0, 6.8Hz, 1H), 2.30(ddd, J=9.8, 6.4, 1.8Hz, 1H), 2.67(t, J=7.0Hz, 2H), 3.24(s, 2H), 3.75(s, 3H), 3.92-3.98(m, 1H), 4.32-4.41(m, 1H), 4.43-4.51(m, 1H).
   IR(neat):
      3401, 2925, 2852, 2233, 1737, 1448, 1283, 1136, 1089, 1044, 895cm⁻¹.
(5) Following a manner similar to that of Example 1(6) using the compound obtained in (4), 3-thia-9-deoxy-9β-chloro-17,18,19,20-tetranor-16-cyclohexyl-13,14-didehydro-PGF₁α was obtained.
   ¹H-NMR(CDCl₃, 300MHz) δ ppm; 0.85-1.04(m, 2H), 1.08-1.82(m, 17H), 2.09-2.36(m, 1H), 2.19(ddd, J=14.0, 7.7, 6.3Hz, 1H), 2.27(dt, J=14.0, 6.7Hz, 1H), 2.32(ddd, J=9.9, 6.5, 1.6Hz, 1H), 2.62-2.79(m, 2H), 3.24(s, 2H), 3.90-4.00(m, 1H), 4.30-4.55(m, 2H).
   IR(neat):
      3368, 2925, 2852, 2236, 1713, 1448, 1279, 1135, 1089, 1044, 981, 679cm⁻¹.

### Example 11

### Preparations of (17S)-3-thia-9-deoxy-9β-chloro-17,20-dimethyl-13,14-didehydro-PGF₁α and methyl ester thereof

(1) Following a manner similar to that of Example 1(2) using (3R,4R)-2-methylene-3-[(3'S,5'S)-3'-(t-butyldimethylsiloxy)-5'-methylnonan-1'-ynyl]-4-(t-butyldimethylsiloxy)cyclopentan-1-one, (17S)-3-thia-17,20-dimethyl-13,14-didehydro-PGE₁ methyl ester 11,15-bis(t-butyldimethylsilyl ether) was obtained.
   ¹H-NMR(CDCl₃, 200MHz) δ ppm; 0.09(s, 6H), 0.11(s, 3H), 0.12(s, 3H), 0.84-0.94(m, 6H), 0.89(s, 9H), 0.90(s, 9H), 0.96-1.86(m, 15H), 2.07-2.27(m, 1H), 2.17(dd, J=18.3, 6.9Hz, 1H), 2.48-2.76(m, 4H), 3.22(s, 2H), 3.74(s, 3H), 4.22-4.36(m, 1H), 4.38-4.50(m, 1H).
   IR(neat):
      2955, 2930, 2858, 2233, 1748, 1463, 1437, 1408, 1379, 1362, 1279, 1256, 1131, 1093, 1007, 940, 838, 778, 670cm⁻¹.
(2) Following a manner similar to that of Example 1(3) using the compound obtained in (1), (17S)-3-thia-17,20-dimethyl-13,14-didehydro-PGF₁α methyl ester 11,15-bis(t-butyldimethylsilyl ether) and (17S)-3-thia-17,20-dimethyl-13,14-didehydro-PGF₁β methyl ester 11,15-bis(t-butyldimethylsilyl ether) were obtained.
   (17S)-3-Thia-17,20-dimethyl-13,14-didehydro-PGF₁α methyl ester 11,15-bis(t-butyldimethylsilyl ether)
   ¹H-NMR(CDCl₃, 200MHz) δ ppm; 0.09(s, 6H), 0.10(s, 3H), 0.11(s, 3H), 0.82-0.94(m, 6H), 0.88(s, 9H), 0.90(s, 9H), 1.06-2.12(m, 18H), 2.38-2.49(m, 1H), 2.65(t, J=6.8Hz, 2H), 3.23(s, 2H), 3.74(s, 3H), 4.05-4.20(m, 1H), 4.22-4.32(m, 1H), 4.35-4.48(m, 1H).
   IR(neat):
      3523, 2955, 2930, 2858, 2230, 1741, 1463, 1437, 1388, 1362, 1256, 1129, 1084, 1006, 939, 870, 838, 778, 668cm⁻¹.

   (17S)-3-Thia-17,20-dimethyl-13,14-didehydro-PGF₁β methyl ester 11,15-bis(t-butyldimethylsilyl ether)
   ¹H-NMR(CDCl₃, 200MHz) δ ppm; 0.07(s, 3H), 0.09(s, 3H), 0.10(s, 3H), 0.12(s, 3H), 0.80-0.93(m, 6H), 0.86(s, 9H), 0.90(s, 9H), 0.96-1.96(m, 18H), 2.22(ddd, J=9.2, 6.2, 1.6Hz, 1H), 2.64(t, J=6.9Hz, 2H), 3.22(s, 2H), 3.74(s, 3H), 3.91-4.05(m, 1H), 4.15-4.29(m, 1H), 4.42(dt, J=1.6, 7.0Hz, 1H).
   IR(neat):
      3468, 2955, 2930, 2858, 2231, 1740, 1463, 1438, 1385, 1362, 1281, 1255, 1130, 1072, 1006, 837, 778, 669cm⁻¹.
(3) Following a manner similar to that of Example 1(4) using (17S)-3-thia-17,20-dimethyl-13,14-didehydro-PGF₁α methyl ester 11,15-bis(t-butyldimethylsilyl ether) obtained in (2), (17S)-3-thia-9-deoxy-9β-chloro-17,20-dimethyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.10(s, 3H), 0.12(s, 3H), 0.84-0.94(m, 6H), 0.88(s, 9H), 0.90(s, 9H), 1.04-1.77(m, 15H), 1.96-2.19(m, 3H), 2.28(ddd, J=8.8, 4.8, 1.7Hz, 1H), 2.65(t, J=7.1Hz, 2H), 3.23(s, 2H), 3.74(s, 3H), 3.87-4.03(m, 1H), 4.18-4.29(m, 1H), 4.42(dt, J=1.7, 7.0Hz, 1H).
   IR(neat):
      2955, 2930, 2858, 2233, 1741, 1463, 1437, 1385, 1362, 1279, 1256, 1129, 1089, 1006, 939, 906, 837, 778, 669cm⁻¹.
(4) Following a manner similar to that of Example 1(5) using the compound obtained in (3), (17S)-3-thia-9-deoxy-9β-chloro-17,20-dimethyl-13,14-didehydro-PGF₁α methyl ester was obtained.
   ¹H-NMR(CDCl₃, 300MHz) δ ppm; 0.84-0.97(m, 3H), 0.91(d, J=6.6Hz, 3H), 1.09-1.75(m, 15H), 2.08-2.22(m, 1H), 2.19(ddd, J=14.0, 7.7, 6.1Hz, 1H), 2.27(dt, J=14.0, 6.8Hz, 1H), 2.30(ddd, J=9.9, 6.4, 1.8Hz, 1H), 2.66(t, J=7.0Hz, 2H), 3.24(s, 2H), 3.75(s, 3H), 3.90-4.00(m, 1H), 4.31-4.49(m, 2H).
   IR(neat):
      3401, 2954, 2929, 2858, 2233, 1738, 1438, 1384, 1282, 1134, 1020, 597cm⁻¹.
(5) Following a manner similar to that of Example 1(6) using the compound obtained in (4), (17S)-3-thia-9-deoxy-9β-chloro-17,20-dimethyl-13,14-didehydro-PGF₁α was obtained.
   ¹H-NMR(CDCl₃, 300MHz) δ ppm; 0.83-0.99(m, 3H), 0.91(d, J=6.0Hz, 3H), 1.09-1.78(m, 15H), 2.08-2.36(m, 1H), 2.20(ddd, J=14.1, 7.8, 6.4Hz, 1H), 2.27(dt, J=14.1, 6.8Hz, 1H), 2.32(ddd, J=9.7, 6.6, 1.7Hz, 1H), 2.63-2.77(m, 2H), 3.24(s, 2H), 3.90-4.00(m, 1H), 4.31-4.40(m, 1H), 4.48(dt, J=1.7, 7.7Hz, 1H), 4.90(br s, 3H).
   IR(neat):
      3350, 2954, 2929, 2858, 2236, 1713, 1460, 1380, 1284, 1135, 1044, 680cm⁻¹.

### Example 12

### Preparations of (17S)-3-thia-9-deoxy-9β-chloro-20-isopropylidene-17-methyl-13,14-didehydro-PGF₁α and methyl ester thereof

(1) Following a manner similar to that of Example 1(2) using (3R,4R)-2-methylene-3-[(3'S,5'S)-3'-(t-butyldimethylsiloxy)-5',9'-dimethyldec-8'-en-1'-ynyl]-4-(t-butyldimethylsiloxy)cyclopentan-1-one, (17S)-3-thia-20-isopropylidene-17-methyl-13,14-didehydro-PGE₁ methyl ester 11,15-bis(t-butyldimethylsilyl ether) was obtained.
   ¹H-NMR(CDCl₃, 200MHz) δ ppm; 0.09(s, 3H), 0.10(s, 3H), 0.11(s, 3H), 0.13(s, 3H), 0.84-0.92(m, 3H), 0.89(s, 9H), 0.90(s, 9H), 1.05-2.08(m, 13H), 1.60(s, 3H), 1.68(d, J=0.9Hz, 3H), 2.10-2.24(m, 1H), 2.17(dd, J=6.5, 18.2Hz, 1H), 2.57-2.74(m, 4H), 3.21(s, 2H), 3.74(s, 3H), 4.23-4.34(m, 1H), 4.36-4.48(m, 1H), 5.03-5.14(m, 1H).
   IR(neat):
      2954, 2930, 2857, 2235, 1747, 1463, 1437, 1377, 1362, 1279, 1255, 1131, 1099, 1074, 1007, 939, 838, 778, 670cm⁻¹.
(2) Following a manner similar to that of Example 1(3) using the compound obtained in (1), (17S)-3-thia-20-isopropylidene-17-methyl-13,14-didehydro-PGF₁α methyl ester 11,15-bis(t-butyldimethylsilyl ether) and (17S)-3-thia-20-isopropylidene-17-methyl-13,14-didehydro-PGF₁β methyl ester 11,15-bis(t-butyldimethylsilyl ether) were obtained.
   (17S)-3-Thia-20-isopropylidene-17-methyl-13,14-didehydro-PGF₁α methyl ester 11,15-bis(t-butyldimethylsilyl ether)
   ¹H-NMR(CDCl₃, 200MHz) δ ppm; 0.10(s, 3H), 0.11(s, 6H), 0.12(s, 3H), 0.85-0.94(m, 3H), 0.89(s, 9H), 0.91(s, 9H), 1.06-2.14(m, 16H), 1.60(s, 3H), 1.68(s, 3H), 2.39-2.48(m, 1H), 2.65(t, J=7.0Hz, 2H), 3.23(s, 2H), 3.74(s, 3H), 4.07-4.16(m, 1H), 4.22-4.31(m, 1H), 4.42(dt, J=1.9, 7.1Hz, 1H), 5.03-5.16(m, 1H).
   IR(neat):
      3524, 2954, 2930, 2857, 2231, 1740, 1472, 1463, 1437, 1378, 1362, 1278, 1256, 1100, 1072, 1006, 939, 905, 838, 778, 668cm⁻¹.

   (17S)-3-Thia-20-isopropylidene-17-methyl-13,14-didehydro-PGF₁β methyl ester 11,15-bis(t-butyldimethylsilyl ether)
   ¹H-NMR(CDCl₃, 200MHz) δ ppm; 0.07(s, 3H), 0.09(s, 3H), 0.10(s, 3H), 0.12(s, 3H), 0.84-0.93(m, 3H), 0.88(s, 9H), 0.90(s, 9H), 1.04-2.07(m, 16H), 1.60(s, 3H), 1.68(s, 3H), 2.21(ddd, J=9.3, 6.2, 1.7Hz, 1H), 2.64(t, J=6.8Hz, 2H), 3.22(s, 2H), 3.74(s, 3H), 3.90-4.05(m, 1H), 4.14-4.29(m, 1H), 4.33-4.48(m, 1H), 5.02-5.16(m, 1H).
   IR(neat):
      3467, 2955, 2929, 2857, 2232, 1739, 1472, 1463, 1438, 1384, 1362, 1281, 1255, 1131, 1071, 1006, 907, 837, 778, 669cm⁻¹.
(3) Following a manner similar to that of Example 1(4) using (17S)-3-thia-20-isopropylidene-17-methyl-13,14-didehydro-PGF₁α methyl ester 11,15-bis(t-butyldimethylsilyl ether) obtained in (2), (17S)-3-thia-9-deoxy-9β-chloro-20-isopropylidene-17-methyl-13,14-didehydro-PGF₁α methyl ester 11,15-bis(t-butyldimethylsilyl ether) was obtained.
   ¹H-NMR (CDCl₃, 200MHz) δ ppm; 0.07(s, 3H), 0.09(s, 3H), 0.10(s, 3H), 0.12(s, 3H), 0.85-0.94(m, 3H), 0.88(s, 9H), 0.90(s, 9H), 1.05-1.82(m, 11H), 1.60(s, 3H), 1.68(s, 3H), 1.88-2.19(m, 5H), 2.27(ddd, J=8.9, 4.8, 1.8Hz, 1H), 2.64(t, J=7.0Hz, 2H), 3.23(s, 2H), 3.74(s, 3H), 3.87-4.03(m, 1H), 4.19-4.30(m, 1H), 4.42(dt, J=1.8, 7.1Hz, 1H), 5.04-5.17(m, 1H).
   IR(neat):
      2954, 2930, 2857, 2234, 1741, 1472, 1463, 1437, 1384, 1362, 1279, 1256, 1130, 1093, 1073, 1007, 939, 907, 837, 811, 778, 669cm⁻¹.
(4) Following a manner similar to that of Example 1(5) using the compound obtained in (3), (17S)-3-thia-9-deoxy-9β-chloro-20-isopropylidene-17-methyl-13,14-didehydro-PGF₁α methyl ester was obtained.
   ¹H-NMR(CDCl₃, 300MHz) δ ppm; 0.93(d, J=6.3Hz, 3H), 1.13-1.75(m, 11H), 1.61(s, 3H), 1.69(s, 3H), 1.88-2.33(m, 3H), 2.22(ddd, J=14.0, 7.7, 6.2Hz, 1H), 2.27(dt, J=14.0, 6.8Hz, 1H), 2.29(ddd, J=9.9, 6.4, 1.8Hz, 1H), 2.66(t, J=6.9Hz, 2H), 3.23(s, 2H), 3.75(s, 3H), 3.89-4.00(m, 1H), 4.31-4.41(m, 1H), 4.45(dt, J=1.8, 7.5Hz, 1H), 5.05-5.15(m, 1H).
   IR(neat):
      3401, 2928, 2856, 2234, 1738, 1438, 1379, 1283, 1136, 1089, 1028, 742cm⁻¹.
(5) Following a manner similar to that of Example 1(6) using the compound obtained in (4), (17S)-3-thia-9-deoxy-9β-chloro-20-isopropylidene-17-methyl-13,14-didehydro-PGF₁α was obtained.
   ¹H-NMR(CDCl₃, 300MHz) δ ppm; 0.93(d, J=6.4Hz, 3H), 1.12-1.82(m, 11H), 1.61(s, 3H), 1.69(s, 3H), 1.88-2.36(m, 3H), 2.19(ddd, J=14.0, 7.7, 6.3Hz, 1H), 2.27(dt, J=14.0, 6.9Hz, 1H), 2.32(ddd, J=9.9, 6.4, 1.8Hz, 1H), 2.63-2.76(m, 2H), 3.24(s, 2H), 3.90-4.00(m, 1H), 4.30-4.41(m, 1H), 4.49(dt, J=1.8, 7.1Hz, 1H), 5.06-5.19(m, 1H).
   IR(neat):
      3369, 2929, 2857, 2236, 1713, 1445, 1380, 1284, 1136, 1088, 1031, 680cm⁻¹.

### Example 13

### Preparations of 3-thia-9-deoxy-9β-bromo-16,17,18,19,20-pentanor-15-cyclohexyl-13,14-didehydro-PGF₁α and methyl ester thereof

(1) To a solution of 3-thia-16,17,18,19,20-pentanor-15-cyclohexyl-13,14-didehydro-PGF₁α methyl ester 11,15-bis(t-butyldimethylsilyl ether) obtained in Example 1(3) (174 mg, 0.2775 mmol) in acetonitrile (2.8 ml) were added pyridine (0.045 ml, 0.555 mmol), carbon tetrabromide (184 mg, 0.5655 mmol) and triphenylphosphin (109 mg, 0.416 mmol), followed by stirring at room temperature for 5 hours. The solution, after addition of hexane - ether (1/1, solution), was washed with water and a saturated aqueous sodium chloride solution and dried. After concentration, the resulting crude product was purified by silica gel chromatography (eluent; n-hexane : AcOEt = 10:1) to give 153 mg (0.227 mmol) of 3-thia-9-deoxy-9β-bromo-16,17,18,19,20-pentanor-15-cyclohexyl-13,14-didehydro-PGF₁α methyl ester 11,15-bis(t-butyldimethylsilyl ether).
   ¹H-NMR(CDCl₃, 200MHz) δ ppm; 0.07(s, 3H), 0.08(s, 3H), 0.10(s, 3H), 0.11(s, 3H), 0.73-1.92(m, 18H), 0.87(s, 9H), 0.90(s, 9H), 2.08-2.39(m, 3H), 2.54-2.70(m, 2H), 3.23(s, 2H), 3.74(s, 3H), 3.88-4.02(m, 1H), 4.07(dd, J=6.0, 1.5Hz, 1H), 4.18-4.28(m, 1H).
   IR(neat):
      2929, 2856, 2232, 1741, 1472, 1463, 1437, 1362, 1278, 1255, 1101, 1072, 1007, 899, 838, 778, 670cm⁻¹.
(2) Following a manner similar to that of Example 1(5) using the compound obtained in (1), 3-thia-9-deoxy-9β-bromo-16,17,18,19,20-pentanor-15-cyclohexyl-13,14-didehydro-PGF₁α methyl ester was obtained.
   ¹H-NMR(CDCl₃, 300MHz) δ ppm; 0.94-1.90(m, 18H), 2.12-2.46(m, 3H), 2.58-2.72(m, 2H), 3.23(s, 2H), 3.75(s, 3H), 3.93-4.02(m, 1H), 4.16(dd, J=5.9, 1.3Hz, 1H), 4.31-4.42(m, 1H).
   IR(neat):
      3401, 2927, 2853, 2235, 1736, 1438, 1282, 1139, 1084, 1011, 894, 690cm⁻¹.
(3) Following a manner similar to that of Example 1(6) using the compound obtained in (2), 3-thia-9-deoxy-9β-bromo-16,17,18,19,20-pentanor-15-cyclohexyl-13,14-didehydro-PGF₁α was obtained.
   ¹H-NMR(CDCl₃, 300MHz) δ ppm; 0.94-1.37(m, 6H), 1.42-1.96(m, 12H), 2.13-2.49(m, 3H), 2.60-2.84(m, 2H), 3.24(s, 2H), 3.92-4.05(m, 1H), 4.20(dd, J=6.1, 1.6, 1H), 4.33-4.43(m, 1H).
   IR(KBr):
      3369, 2927, 2854, 2222, 1715, 1447, 1418, 1297, 1213, 1173, 1143, 1085, 1050, 1008, 960, 892, 683cm⁻¹.

### Example 14

### Preparations of 3-thia-9-deoxy-9α-bromo-16,17,18,19,20-pentanor-15-cyclohexyl-13,14-didehydro-PGF₁α and methyl ester thereof

(1) Following a manner similar to that of Example 13(1) using 3-thia-16,17,18,19,20-pentanor-15-cyclohexyl-13,14-didehydro-PGF₁β methyl ester 11,15-bis(t-butyldimethylsilyl ether) obtained in Example 1(3), 3-thia-9-deoxy-9α-bromo-16,17,18,19,20-pentanor-15-cyclohexyl-13,14-didehydro-PGF₁α methyl ester 11,15-bis(t-butyldimethylsilyl ether) was obtained.
   ¹H-NMR(CDCl₃, 200MHz) δ ppm; 0.06(s, 3H), 0.08(s, 3H), 0.09(s, 3H), 0.10(s, 3H), 0.73-1.94(m, 18H), 0.89(s, 9H), 0.90(s, 9H), 2.22(ddd, J=15.6, 3.3, 1.8Hz, 1H), 2.52-2.82(m, 4H), 3.23(s, 2H), 3.74(s, 3H), 4.09(dd, J=6.2, 1.7Hz, 1H), 4.23(ddd, J=8.2, 6.5, 3.2Hz, 1H), 4.34-4.44(m, 1H).
   IR(neat):
      2929, 2856, 2232, 1741, 1472, 1463, 1388, 1361, 1338, 1278, 1255, 1218, 1101, 1072, 1007, 886, 838, 778, 669cm⁻¹.
(2) Following a manner similar to that of Example 1(5) using the compound obtained in (1), 3-thia-9-deoxy-9α-bromo-16,17,18,19,20-pentanor-15-cyclohexyl-13,14-didehydro-PGF₁α methyl ester was obtained.
   ¹H-NMR(CDCl₃, 300MHz) δ ppm; 0.96-1.94(m, 18H), 2.35(ddd, J=16.1, 2.8, 1.1Hz, 1H), 2.58-2.87(m, 3H), 2.82(ddd, J=16.1, 8.7, 5.7, 1H), 3.23(s, 2H), 3.75(s, 3H), 4.17(dd, J=6.0, 1.7Hz, 1H), 4.27-4.37(m, 1H), 4.42-4.49(m, 1H).
   IR(neat):
      3401, 2927, 2853, 2236, 1735, 1437, 1280, 1217, 1137, 1085, 1011, 894, 830, 690, 598cm⁻¹.
(3) Following a manner similar to that of Example 1(6) using the compound obtained in (2), 3-thia-9-deoxy-9α-bromo-16,17,18,19,20-pentanor-15-cyclohexyl-13,14-didehydro-PGF₁α was obtained.
   ¹H-NMR(CDCl₃, 300MHz) δ ppm; 0.94-1.92(m, 18H), 2.31-2.42(m, 1H), 2.61-2.90(m, 3H), 2.83(ddd, J=16.0, 8.6, 5.7Hz, 1H), 3.22(s, 2H), 4.24(dd, J=6.1, 2.1Hz, 1H), 4.26-4.36(m, 1H), 4.42-4.50(m, 1H).
   IR(KBr):
      3401, 2930, 2856, 2239, 1706, 1458, 1415, 1344, 1303, 1275, 1213, 1140, 1107, 1084, 1063, 1040, 1002, 889, 830, 721, 680, 591cm⁻¹.

### Example 15

### Preparations of 3-thia-9-deoxy-9β-bromo-16,17,18,19,20-pentanor-15-cyclopentyl-13,14-didehydro-PGF₁α and methyl ester thereof

(1) Following a manner similar to that of Example 13(1) using 3-thia-16,17,18,19,20-pentanor-15-cyclopentyl-13,14-didehydro-PGF₁α methyl ester 11,15-bis(t-butyldimethylsilyl ether) obtained in Example 6(2), 3-thia-9-deoxy-9β-bromo-16,17,18,19,20-pentanor-15-cyclopentyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.09(s, 6H), 0.12(s, 3H), 0.87(s, 9H), 0.90(s, 9H), 1.17-1.79(m, 15H), 2.04-2.33(m, 4H), 2.65(t, J=7.0Hz, 2H), 3.23(s, 2H), 3.74(s, 3H), 3.90-4.04(m, 1H), 4.15-4.26(m, 1H), 4.17(dd, J=6.9, 1.6Hz, 1H).
   IR(neat):
      2954, 2931, 2858, 2231, 1741, 1472, 1463, 1437, 1388, 1362, 1278, 1256, 1073, 1007, 940, 906, 838, 778, 671cm⁻¹.
(2) Following a manner similar to that of Example 1(5) using the compound obtained (1), 3-thia-9-deoxy-9β-bromo-16,17,18,19,20-pentanor-15-cyclopentyl-13,14-didehydro-PGF₁α methyl ester was obtained.
   ¹H-NMR(CDCl₃, 300MHz) δ ppm; 1.34-1.93(m, 14H), 2.09-2.44(m, 3H), 2.27(ddd, J=14.1, 7.8, 5.6Hz, 1H), 2.40(dt, J=14.1, 7.1Hz, 1H), 2.66(t, J=6.9Hz, 2H), 3.24(s, 2H), 3.75(s, 3H), 3.93-4.01(m, 1H), 4.25(dd, J=7.0, 1.4Hz, 1H), 4.32-4.41(m, 1H).
   IR(neat):
      3401, 2950, 2864, 2233, 1735, 1438, 1385, 1283, 1138, 1026, 594cm⁻¹.
(3) Following a manner similar to that of Example 1(6) using the compound obtained in (2), 3-thia-9-deoxy-9β-bromo-16,17,18,19,20-pentanor-15-cyclopentyl-13,14-didehydro-PGF₁α was obtained.
   ¹H-NMR(CDCl₃, 300MHz) δ ppm; 1.18-1.86(m, 14H), 2.08-2.46(m, 3H), 2.28(ddd, J=14.2, 7.7, 5.7Hz, 1H), 2.40(dt, J=14.2, 7.1Hz, 1H), 2.52-2.86(m, 2H), 3.28(br s, 2H), 3.92-4.02(m, 1H), 4.28(dd, J=7.2, 1.6Hz, 1H), 4.30-4.42(m, 1H), 4.48(br s, 3H).
   IR(KBr):
      3323, 2947, 2853, 2221, 1712, 1679, 1459, 1415, 1387, 1355, 1300, 1246, 1215, 1174, 1141, 1087, 1030, 953, 866, 759, 726, 677, 581cm⁻¹.

### Example 16

### Preparations of 3-thia-9-deoxy-9β-bromo-16,17,18,19,20-pentanor-15-cycloheptyl-13,14-didehydro-PGF₁α and methyl ester thereof

(1) Following a manner similar to that of Example 13(1) using 3-thia-16,17,18,19,20-pentanor-15-cycloheptyl-13,14-didehydro-PGF₁α methyl ester 11,15-bis(t-butyldimethylsilyl ether) obtained in Example 7(2), 3-thia-9-deoxy-9β-bromo-16,17,18,19,20-pentanor-15-cycloheptyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.09(s, 3H), 0.11(s, 3H), 0.88(s, 9H), 0.90(s, 9H), 1.16-1.92(m, 19H), 2.07-2.36(m, 4H), 2.65(t, J=7.1Hz, 2H), 3.23(s, 2H), 3.74(s, 3H), 3.90-4.06(m, 1H), 4.11-4.28(m, 2H).
   IR(neat):
      2929, 2857, 2230, 1741, 1472, 1463, 1437, 1408, 1387, 1362, 1278, 1255, 1130, 1084, 1007, 940, 837, 777, 671cm⁻¹.
(2) Following a manner similar to that of Example 1(5) using the compound obtained in (1), 3-thia-9-deoxy-9β-bromo-16,17,18,19,20-pentanor-15-cycloheptyl-13,14-didehydro-PGF₁α methyl ester was obtained.
   ¹H-NMR(CDCl₃, 300MHz) δ ppm; 1.21-1.92(m, 19H), 2.21-2.36(m, 2H), 2.27(ddd, J=14.1, 7.8, 5.7Hz, 1H), 2.40(dt, J=14.1, 7.1Hz, 1H), 2.66(t, J=7.0Hz, 2H), 3.24(s, 2H), 3.75(s, 3H), 3.92-4.03(m, 1H), 4.23(dd, J=5.3, 1.4Hz, 1H), 4.32-4.42(m, 1H).
   IR(neat):
      3401, 2926, 2855, 2232, 1736, 1438, 1282, 1137, 1048, 1013cm⁻¹.
(3) Following a manner similar to that of Example 1(6) using the compound obtained in (2), 3-thia-9-deoxy-9β-bromo-16,17,18,19,20-pentanor-15-cycloheptyl-13,14-didehydro-PGF₁α was obtained.
   ¹H-NMR(CDCl₃, 300MHz) δ ppm; 1.16-1.93(m, 19H), 2.20-2.45(m, 2H), 2.28(ddd, J=14.2, 7.7, 5.8Hz, 1H), 2.40(dt, J=14.2, 7.1Hz, 1H), 2.61-2.80(m, 2H), 3.24(s, 2H), 3.70(br s, 3H), 3.93-4.03(m, 1H), 4.26(dd, J=5.4, 1.6Hz, 1H), 4.33-4.41(m, 1H).
   IR(neat):
      3369, 2926, 2856, 2234, 1713, 1446, 1280, 1142, 1047, 680cm⁻¹

### Example 17

### Preparations of (2'RS)-3-thia-9-deoxy-9β-bromo-16,17,18,19,20-pentanor-15-(2'-methylcyclohexyl)-13,14-didehydro-PGF₁α and methyl ester thereof

(1) Following a manner similar to that of Example 13(1) using 3-thia-16,17,18,19,20-pentanor-15-(2'-methylcyclohexyl)-13,14-didehydro-PGF₁α methyl ester 11,15-bis(t-butyldimethylsilyl ether) obtained in Example 8(2), (2'RS)-3-thia-9-deoxy-9β-bromo-16,17,18,19,20-pentanor-15-(2'-methylcyclohexyl)-13,14-didehydro-PGF₁α methyl ester 11,15-bis(t-butylmethylsilyl ether) was obtained.
   ¹H-NMR(CDCl₃, 200MHz) δ ppm; 0.07, 0.08, 0.09, 0.10, 0.12 and 0.15(6s, 12H), 0.84 and 0.90(2d, J=7.0Hz, 3H), 0.88, 0.89 and 0.91(3s, 18H), 1.07-1.82(m, 16H), 2.08-2.38(m, 4H), 2.65(t, J=6.8Hz, 2H), 3.23(s, 2H), 3.74(s, 3H), 3.90-4.10(m, 2H), 4.17-4.29(m, 1H).
   IR(neat):
      2953, 2929, 2857, 2230, 1741, 1472, 1463, 1437, 1385, 1362, 1278, 1256, 1101, 1064, 1006, 939, 906, 838, 778, 669cm⁻¹.
(2) Following a manner similar to that of Example 1(5) using the compound obtained in (1), (2'RS)-3-thia-9-deoxy-9β-bromo-16,17,18,19,20-pentanor-15-(2'-methylcyclohexyl)-13,14-didehydro-PGF₁α methyl ester was obtained.
   ¹H-NMR(CDCl₃, 300MHz) δ ppm; 0.88 and 0.91(2d, J=7.3Hz, 3H), 1.12-1.85(m, 15H), 2.06-2.45(m, 4H), 2.40(dt, J=14.1, 7.1Hz, 1H), 2.66(t, J=6.8Hz, 2H), 3.24(s, 2H), 3.75(s, 3H), 3.92-4.16(m, 2H), 4.33-4.41(m, 1H).
   IR(neat):
      3401, 2926, 2856, 2232, 1735, 1438, 1384, 1281, 1217, 1135, 1085, 1017, 978, 757cm⁻¹.
(3) Following a manner similar to that of Example 1(6) using the compound obtained in (2), (2'RS)-3-thia-9-deoxy-9β-bromo-16,17,18,19,20-pentanor-15-(2'-methylcyclohexyl)-13,14-didehydro-PGF₁α was obtained.
   ¹H-NMR(CDCl₃, 300MHz) δ ppm; 0.88 and 0.91(2d, J=6.8Hz 3H), 1.09-1.85(m, 15H), 2.10-2.46(m, 3H), 2.28(ddd, J=14.1, 7.8, 5.9Hz, 1H), 2.40(dt, J=14.1, 7.1Hz, 1H), 2.71(t, J=6.4Hz, 2H), 3.24(s, 2H), 3.54(br s, 3H), 3.93-4.03(m, 1H), 4.07-4.19(m, 1H), 4.31-4.42(m, 1H).
   IR(neat):
      3369, 2927, 2857, 2234, 1713, 1447, 1383, 1283, 1217, 1143, 1086, 1019, 977, 758, 668cm⁻¹.

### Example 18

### Preparations of 3-thia-9-deoxy-9β-bromo-17,18,19,20-tetranor-16-cyclopentyl-13,14-didehydro-PGF₁α and methyl ester thereof

(1) Following a manner similar to that of Example 13(1) using 3-thia-17,18,19,20-tetranor-16-cyclopentyl-13,14-didehydro-PGF₁α methyl ester 11,15-bis(t-butyldimethylsilyl ether) obtained in Example 9(2), 3-thia-9-deoxy-9β-bromo-17,18,19,20-tetranor-16-cyclopentyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.10(s, 3H), 0.12(s, 3H), 0.87(s, 9H), 0.90(s, 9H), 0.98-2.37(m, 21H), 2.65(t, J=7.0Hz, 2H), 3.23(s, 2H), 3.74(s, 3H), 3.90-4.05(m, 1H), 4.18-4.29(m, 1H), 4.35(dt, J=1.3, 6.8Hz, 1H).
   IR(neat):
      2952, 2931, 2857, 2232, 1741, 1472, 1463, 1437, 1387, 1362, 1278, 1255, 1131, 1074, 1007, 940, 905, 837, 778, 670cm⁻¹.
(2) Following a manner similar to that of Example 1(5) using the compound obtained in (1), 3-thia-9-deoxy-9β-bromo-17,18,19,20-tetranor-16-cyclopentyl-13,14-didehydro-PGF₁α methyl ester was obtained.
   ¹H-NMR(CDCl₃, 300MHz) δ ppm; 1.05-1.22(m, 2H), 1.46-2.06(m, 15H), 2.20-2.42(m, 2H), 2.27(ddd, J=14.2, 7.7, 5.6Hz, 1H), 2.40(dt, J=14.2, 7.1Hz, 1H), 2.59-2.73(m, 2H), 3.24(s, 2H), 3.75(s, 3H), 3.92-4.02(m, 1H), 4.33-4.42(m, 2H).
   IR(neat):
      3400, 2946, 2860, 2233, 1737, 1437, 1385, 1283, 1137, 1044, 1014, 594cm⁻¹.
(3) Following a manner similar to that of Example 1(6) using the compound obtained in (2), 3-thia-9-deoxy-9β-bromo-17,18,19,20-tetranor-16-cyclopentyl-13,14-didehydro-PGF₁α was obtained.
   ¹H-NMR(CDCl₃, 300MHz) δ ppm; 1.05-1.32(m, 2H), 1.46-2.06(m, 15H), 2.12-2.46(m, 2H), 2.28(ddd, J=14.2, 7.8, 5.7Hz, 1H), 2.40(dt, J=14.2, 7.0Hz, 1H), 2.56-2.89(m, 2H), 3.23(br s, 2H), 3.92-4.03(m, 1H), 4.20(br s, 3H), 4.30-4.50(m, 2H).
   IR(neat):
      3369, 2942, 2960, 2235, 1713, 1419, 1281, 1142, 1044, 676cm⁻¹

### Example 19

### Preparations of 3-thia-9-deoxy-9β-bromo-17,18,19,20-tetranor-16-cyclohexyl-13,14-didehydro-PGF₁α and methyl ester thereof

(1) Following a manner similar to that of Example 13(1) using 3-thia-17,18,19,20-tetranor-16-cyclohexyl-13,14-didehydro-PGF₁α methyl ester 11,15-bis(t-butyldimethylsilyl ether) obtained in Example 10(2), 3-thia-9-deoxy-9β-bromo-17,18,19,20-tetranor-16-cyclohexyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.09(s, 3H), 0.10(s, 3H), 0.12(s, 3H), 0.73-1.80(m, 19H), 0.88(s, 9H), 0.90(s, 9H), 2.08-2.37(m, 4H), 2.65(t, J=7.0Hz, 2H), 3.23(s, 2H), 3.74(s, 3H), 3.91-4.06(m, 1H), 4.17-4.29(m, 1H), 4.35-4.50(m, 1H).
   IR(neat):
      2928, 2856, 2232, 1741, 1472, 1463, 1448, 1387, 1362, 1278, 1255, 1129, 1095, 1074, 1006, 939, 837, 778, 669cm⁻¹.
(2) Following a manner similar to that of Example 1(5) using the compound obtained in (1), 3-thia-9-deoxy-9β-bromo-17,18,19,20-tetranor-16-cyclohexyl-13,14-didehydro-PGF₁α methyl ester was obtained.
   ¹H-NMR(CDCl₃, 300MHz) δ ppm; 0.85-1.04(m, 2H), 1.07-1.34(m, 4H), 1.44-1.86(m, 13H), 2.21-2.35(m, 2H), 2.27(ddd, J=14.1, 7.7, 5.6Hz, 1H), 2.40(dt, J=14.1, 7.0Hz, 1H), 2.67(t, J=7.0Hz, 2H), 3.24(s, 2H), 3.75(s, 3H), 3.92-4.02(m, 1H), 4.33-4.41(m, 1H), 4.42-4.50(m, 1H).
   IR(neat):
      3392, 2924, 2851, 2233, 1735, 1448, 1283, 1199, 1137, 1045, 895, 592cm⁻¹.
(3) Following a manner similar to that of Example 1(6) using the compound obtained in (2), 3-thia-9-deoxy-9β-bromo-17,18,19,20-tetranor-16-cyclohexyl-13,14-didehydro-PGF₁α was obtained.
   ¹H-NMR(CDCl₃, 300MHz) δ ppm; 0.85-1.04(m, 2H), 1.08-1.33(m, 4H), 1.42-1.84(m, 13H), 2.20-2.45(m, 2H), 2.28(ddd, J=14.0, 7.6, 5.9Hz, 1H), 2.40(dt, J=14.0, 7.0Hz, 1H), 2.60-2.80(m, 2H), 3.24(br s, 2H), 3.93-4.03(m, 1H), 4.15(br s, 3H), 4.33-4.53(m, 2H).
   IR(neat):
      3369, 2925, 2852, 2235, 1713, 1448, 1376, 1273, 1142, 1044, 982, 895, 677cm⁻¹.

### Example 20

### Preparations of (17S)-3-thia-9-deoxy-9β-bromo-17,20-dimethyl-13,14-didehydro-PGF₁α and methyl ester thereof

(1) Following a manner similar to that of Example 13(1) using (17S)-3-thia-17,20-dimethyl-13,14-didehydro-PGF₁α methyl ester 11,15-bis(t-butyldimethylsilyl ether) obtained in Example 10(2), (17S)-3-thia-9-deoxy-9β-bromo-17,20-dimethyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.10(s, 3H), 0.12(s, 3H), 0.75-0.94(m, 6H), 0.88(s, 9H), 0.90(s, 9H), 1.04-1.76(m, 15H), 2.09-2.37(m, 4H), 2.65(t, J=6.9Hz, 2H), 3.23(s, 2H), 3.74(s, 3H), 3.90-4.05(m, 1H), 4.18-4.28(m, 1H), 4.35-4.48(m, 1H).
   IR(neat):
      2954, 2929, 2857, 2231, 1741, 1463, 1437, 1408, 1385, 1362, 1277, 1255, 1128, 1088, 1007, 939, 905, 837, 777, 668cm⁻¹.
(2) Following a manner similar to that of Example 1(5) using the compound obtained in (1), (17S)-3-thia-9-deoxy-9β-bromo-17,20-dimethyl-13,14-didehydro-PGF₁α methyl ester was obtained.
   ¹H-NMR(CDCl₃, 300MHz) δ ppm; 0.82-0.95(m, 3H), 0.92(d, J=6.4Hz, 3H), 1.09-1.77(m, 15H), 2.20-2.44(m, 2H), 2.27(ddd, J=14.1, 7.7, 5.5Hz, 1H), 2.40(dt, J=14.1, 7.0Hz, 1H), 2.67(t, J=7.0Hz, 2H), 3.24(s, 2H), 3.75(s, 3H), 3.92-4.02(m, 1H), 4.32-4.48(m, 2H).
   IR(neat):
      3400, 2954, 2929, 2857, 2233, 1737, 1438, 1380, 1283, 1196, 1136, 1045, 1014, 730cm⁻¹.
(3) Following a manner similar to that of Example 1(6) using the compound obtained in (2), (17S)-3-thia-9-deoxy-9β-bromo-17,20-dimethyl-13,14-didehydro-PGF₁α was obtained.
   ¹H-NMR(CDCl₃, 300MHz) δ ppm; 0.88-1.00(m, 3H), 0.91(d, J=6.3Hz, 3H), 1.08-1.85(m, 15H), 2.13-2.46(m, 2H), 2.28(ddd, J=14.1, 7.9, 6.0Hz, 1H), 2.40(dt, J=14.1, 7.1Hz, 1H), 2.69(t, J=6.7Hz, 2H), 3.24(s, 2H), 3.93-4.03(m, 1H), 4.22(br s, 3H), 4.30-4.53(m, 2H).
   IR(neat):
      3369, 2954, 2929, 2858, 2234, 1713, 1460, 1380, 1282, 1141, 1045, 679cm⁻¹.

### Example 21

### Preparations of (17S)-3-thia-9-deoxy-9β-bromo-20-isopropylidene-17-methyl-13,14-didehydro-PGF₁α and methyl ester thereof

(1) Following a manner similar to that of Example 13(1) using (17S)-3-thia-9-deoxy-20-isopropylidene-17-methyl-13,14-didehydro-PGF₁α methyl ester 11,15-bis(t-butyldimethylsilyl ether) obtained in Example 10(2), (17S)-3-thia-9-deoxy-9β-bromo-20-isopropylidene-17-methyl-13,14-didehydro-PGF₁α methyl ester 11,15-bis(t-butyldimethylsilyl ether) was obtained.
   ¹H-NMR(CDCl₃, 200MHz) δ ppm; 0.07(s, 3H), 0.08(s, 3H), 0.10(s, 3H), 0.12(s, 3H), 0.77-1.80(m, 14H), 0.87(s, 9H), 0.90(s, 9H), 1.60(s, 3H), 1.68(s, 3H), 1.88-2.06(m, 2H), 2.10-2.36(m, 4H), 2.65(t, J=6.9Hz, 2H), 3.23(s, 2H), 3.74(s, 3H), 3.90-4.04(m, 1H), 4.18-4.28(m, 1H), 4.36-4.47(m, 1H), 5.02-5.16(m, 1H).
   IR(neat):
      2954, 2930, 2857, 2232, 1741, 1472, 1463, 1437, 1384, 1362, 1278, 1256, 1073, 1007, 939, 907, 837, 778, 669cm⁻¹.
(2) Following a manner similar to that of Example 1(5) using the compound obtained in (1), (17S)-3-thia-9-deoxy-9β-bromo-20-isopropylidene-17-methyl-13,14-didehydro-PGF₁α methyl ester was obtained.
   ¹H-NMR(CDCl₃, 300MHz) δ ppm; 0.93(d, J=6.3Hz, 3H), 1.11-1.86(m, 11H), 1.61(s, 3H), 1.69(s, 3H), 1.90-2.08(m, 2H), 2.19-2.34(m, 2H), 2.27(ddd, J=14.2, 7.7, 5.6Hz, 1H), 2.40(dt, J=14.2, 7.1Hz, 1H), 2.66(t, J=7.0Hz, 2H), 3.24(s, 2H), 3.75(s, 3H), 3.91-4.02(m, 1H), 4.34-4.51(m, 2H), 5.06-5.14(m, 1H).
   IR(neat):
      3401, 2928, 2855, 2233, 1736, 1437, 1384, 1281, 1135, 1026, 595cm⁻¹.
(3) Following a manner similar to that of Example 1(6) using the compound obtained in (2), (17S)-3-thia-9-deoxy-9β-bromo-20-isopropylidene-17-methyl-13,14-didehydro-PGF₁α was obtained.
   ¹H-NMR(CDCl₃, 300MHz) δ ppm; 0.93(d, J=6.3Hz, 3H), 1.27-1.83(m, 11H), 1.61(s, 3H), 1.69(s, 3H), 1.91-2.06(m, 2H), 2.20-2.47(m, 2H), 2.28(ddd, J=14.2, 7.7, 6.0Hz, 1H), 2.40(dt, J=14.2, 7.0Hz, 1H), 2.60-2.79(m, 2H), 3.24(br s, 2H), 3.92-4.02(m, 1H), 4.31-4.40(m, 1H), 4.42-4.52(m, 1H), 5.06-5.14(m, 1H).
   IR(neat):
      3368, 2928, 2856, 2235, 1713, 1444, 1379, 1282, 1141, 1087, 1029, 679cm⁻¹.

### Example 22

### Preparations of 3-thia-9-deoxy-9-fluoro-16,17,18,19,20-pentanor-15-cyclohexyl-13,14-didehydro-PGF₁ and methyl ester thereof

(1) To a solution of 3-thia-16,17,18,19,20-pentanor-15-cyclohexyl-13,14-didehydro-PGF₁α methyl ester 11,15-bis(t-butyldimethylsilyl ether) obtained in Example 1(3) (208 mg, 0.332 mmol) in methylene chloride (6.63 ml) was added diethylaminosulfur trifluoride (DAST) (0.438 ml, 3.32 mmol) with stirring at -78°C, and the temperature was elevated to room temperature, followed by stirring for an hour. After addition of ether, the mixture was washed with a saturated aqueous sodium bicarbonate solution and a saturated aqueous sodium chloride solution and dried. After concentration, the resulting crude product was purified by silica gel chromatography (eluent; n-hexane : ethyl acetate = 10:1) to give 3-thia-9-deoxy-9-fluoro-16,17,18,19,20-pentanor-15-cyclohexyl-13,14-didehydro-PGF₁ methyl ester 11,15-bis(t-butyldimethylsilyl ether) (121 mg, 0.192 mmol).
   ¹H-NMR(CDCl₃, 200MHz) δ ppm; 0.07(s, 3H), 0.08(s, 3H), 0.09(s, 3H), 0.10(s, 3H), 0.72-1.99(m, 20H), 0.89(s, 9H), 0.90(s, 9H), 2.47-2.70(m, 3H), 3.23(s, 2H), 3.74(s, 3H), 4.03-4.19(m, 1H), 4.09(dd, J=6.2Hz, 1.4Hz, 1H), 4.69-4.78(m, 1/2H), 4.96-5.04(m, 1/2H).
   IR(neat):
      2930, 2856, 2233, 1741, 1472, 1463, 1387, 1361, 1287, 1255, 1103, 1065, 1007, 899, 838, 778, 670cm⁻¹.
(2) Following a manner similar to that of Example 1(5) using the compound obtained in (1), 3-thia-9-deoxy-9-fluoro-16,17,18,19,20-pentanor-15-cyclohexyl-13,14-didehydro-PGF₁ methyl ester was obtained.
   ¹H-NMR(CDCl₃, 300MHz) δ ppm; 0.93-2.08(m, 20H), 2.24-2.74(m, 3H), 3.23(s, 2H), 3.74(s, 3H), 4.02-4.30(m, 2H), 4.76-5.11(m, 1H).
   IR(KBr):
      3369, 2925, 2852, 2237, 1734, 1449, 1441, 1381, 1346, 1304, 1197, 1170, 1136, 1087, 1057, 1020, 1001, 955, 914, 894, 803, 683, 592cm⁻¹.
(3) Following a manner similar to that of Example 1(5) using the compound obtained in (2), 3-thia-9-deoxy-9-fluoro-16,17,18,19,20-pentanor-15-cyclohexyl-13,14-didehydro-PGF₁ was obtained.
   ¹H-NMR(CDCl₃, 300MHz) δ ppm; 0.70-2.85(m, 23H), 3.12(br s, 3H), 3.24(s, 2H), 4.13-4.36(m, 1H), 4.22(dd, J=6.1Hz, 1.9Hz, 1H), 4.76-4.92(m, 1/2H), 4.96-5.11(m, 1/2H).
   IR(KBr):
      3370, 3171, 2933, 2855, 2239, 1706, 1683, 1447, 1413, 1373, 1345, 1295, 1231, 1222, 1185, 1140, 1112, 1085, 1058, 1017, 987, 946, 893, 803, 674, 574cm⁻¹.

### Example 23

### Preparations of 3-thia-9-deoxy-9β-chloro-16,17,18,19,20-pentanor-15-cyclohexyl-13,14-dihydro-PGF₁α and methyl ester thereof

(1) To a solution of (3R)-1-iodo-3-t-(butyldimethylsiloxy)-3-cyclohexylpropane (5.47 g) in 57.2 ml of ether was added t-butyl lithium (1.7 M, pentane solution, 16.8 ml) at -78°C, followed by stirring for 10 minutes at the same temperature. After addition of lithium 2-thienylcyanocuprate (0.25 M, tetrahydrofuran solution, 66.8 ml), the mixture was stirred for 30 minutes at the same temperature, (4R)-2-(N,N-diethylamino)methyl-4-(t-butyldimethylsiloxy)cyclopent-2- en-1-one (0.25 M, tetrahydrofuran solution, 23.8 ml) was added thereto, and the temperature was elevated to 0°C with stirring. To the reaction solution was added n-hexane (150 ml) - a saturated aqueous ammonium chloride solution (150 ml), the mixture was extracted with n-hexane, and the extract was washed with a saturated aqueous ammonium chloride solution and a saturated aqueous sodium chloride solution, dried and concentrated. The resulting residue was purified by silica gel column chromatography (eluent; n-hexane : AcOEt = 10:1) to give 5.36 g of (3R,4R)-2-methylene-3-[(3'R)-3'-(t-butyldimethylsiloxy)-3'-cyclohexyl-1'-propyl]-4-(t-butyldimethylsiloxy)cyclopentan-1-one.
   ¹H-NMR(CDCl₃, 200MHz) δ ppm; 0.02(s, 3H), 0.03(s, 3H), 0.06(s, 3H), 0.08(s, 3H), 0.55-1.85(m, 15H), 0.88(s, 9H), 0.89(s, 9H), 2.24-2.38(m, 1H), 2.53-2.72(m, 1H), 2.62(dd, J=18.1, 5.9Hz, 1H), 3.37-3.49(m, 1H), 4.06-4.17(m, 1H), 5.27-5.32(m, 1H), 6.08(d, J=2.2Hz, 1H).
   IR(neat):
      2954, 2929, 2856, 1734, 1642, 1473, 1463, 1362, 1256, 1089, 1072, 1006, 939, 836, 775, 670cm⁻¹.
(2) Following a manner similar to that of Example 1(2) using the compound obtained in (1), 3-thia-16,17,18,19,20-pentanor-15-cyclohexyl-13,14-dihydro-PGE₁ methyl ester 11,15-bis(t-butyldimethylsilyl ether) was obtained.
   ¹H-NMR(CDCl₃, 200MHz) δ ppm; 0.03(s, 3H), 0.04(s, 3H), 0.05(s, 3H), 0.08(s, 3H), 0.65-1.95(m, 23H), 0.89(s, 18H), 2.16(dd, J=18.1, 5.9Hz, 1H), 2.47-2.71(m, 3H), 3.22(s, 2H), 3.34-3.47(m, 1H), 3.74(s, 3H), 3.99-4.12(m, 1H).
   IR(neat):
      2929, 2856, 1744, 1472, 1463, 1385, 1278, 1256, 1106, 1072, 1007, 940, 876, 837, 775, 669cm⁻¹.
(3) Following a manner similar to that of Example 1(3) using the compound obtained in (1), 3-thia-16,17,18,19,20-pentanor-15-cyclohexyl-13,14-dihydro-PGF₁α methyl ester 11,15-bis(t-butyldimethylsilyl ether) and 3-thia-16,17,18,19,20-pentanor-15-cyclohexyl-13,14-dihydro-PGF₁β methyl ester 11,15-bis(t-butyldimethylsilyl ether) were obtained.
   3-Thia-16,17,18,19,20-pentanor-15-cyclohexyl-13,14-dihydro-PGF₁α methyl ester 11,15-bis(t-butyldimethylsilyl ether)
   ¹H-NMR(CDCl₃, 200MHz) δ ppm; 0.03(s, 6H), 0.08(s, 6H), 0.75-1.95(m, 25H), 0.88(s, 9H), 0.89(s, 9H), 2.65(t, J=7.1Hz, 2H), 3.23(s, 2H), 3.32-3.43(m, 1H), 3.74(s, 3H), 3.95-4.04(m, 1H), 4.06-4.16(m, 1H).
   IR(neat):
      3523, 2929, 2856, 1741, 1472, 1463, 1436, 1386, 1362, 1278, 1256, 1089, 1072, 1006, 939, 870, 836, 775, 666cm⁻¹.

   3-Thia-16,17,18,19,20-pentanor-15-cyclohexyl-13,14-dihydro-PGF₁β methyl ester 11,15-bis(t-butyldimethylsilyl ether)
   ¹H-NMR(CDCl₃, 200MHz) δ ppm; 0.03 and 0.04(2s, 12H), 0.70-1.95(m, 25H), 0.87(s, 9H), 0.90(s, 9H), 2.64(t, J=7.1Hz, 2H), 3.22(s, 2H), 3.31-3.44(m, 1H), 3.74(s, 3H), 3.87-4.10(m, 2H).
   IR(neat):
      3523, 2929, 2856, 1741, 1472, 1463, 1436, 1386, 1362, 1278, 1256, 1089, 1072, 1006, 939, 870, 836, 775, 666cm⁻¹.
(4) Following a manner similar to that of Example 1(4) using 3-thia-16,17,18,19,20-pentanor-15-cyclohexyl-13,14-dihydro-PGF₁α methyl ester 11,15-bis(t-butyldimethylsilyl ether) obtained in (3), 3-thia-9-deoxy-9β-chloro-16,17,18,19,20-pentanor-15-cyclohexyl-13,14-dihydro-PGF₁α methyl ester 11,15-bis(t-butyldimethylsilyl ether) was obtained.
   ¹H-NMR(CDCl₃, 200MHz) δ ppm; 0.03(s, 3H), 0.04(s, 9H), 0.70-1.84(m, 23H), 0.87(s, 9H), 0.89(s, 9H), 1.96-2.11(m, 2H), 2.56-2.70(m, 2H), 3.23(s, 2H), 3.30-3.43(m, 1H), 3.74(s, 3H), 3.90-4.13(m, 2H).
   IR(neat):
      2929, 2856, 1741, 1472, 1463, 1386, 1361, 1256, 1090, 1072, 1007, 940, 899, 836, 775, 668cm⁻¹.
(5) Following a manner similar to that of Example 1(5) using the compound obtained in (4), 3-thia-9-deoxy-9β-chloro-16,17,18,19,20-pentanor-15-cyclohexyl-13,14-dihydro-PGF₁α methyl ester was obtained.
   ¹H-NMR(CDCl₃, 300MHz) δ ppm; 0.90-1.90(m, 23H), 2.07-2.22(m, 2H), 2.65(t, J=7.1Hz, 2H), 3.23(s, 2H), 3.34-3.43(m, 1H), 3.74(s, 3H), 4.03(dd, J=7.2, 7.0Hz, 1H), 4.05-4.15(m, 1H).
   IR(neat):
      3369, 2927, 2853, 1736, 1450, 1283, 1136, 1011, 893, 699cm⁻¹.
(6) Following a manner similar to that of Example 1(6) using the compound obtained in (5), 3-thia-9-deoxy-9β-chloro-16,17,18,19,20-pentanor-15-cyclohexyl-13,14-dihydro-PGF₁α was obtained.
   ¹H-NMR(CDCl₃, 300MHz) δ ppm; 0.85-1.90(m, 23H), 2.08-2.22(m, 2H), 2.72(t, J=6.6Hz, 2H), 3.20(s, 2H), 3.40-3.55(m, 1H), 3.66(br s, 3H), 4.04(dd, J=7.2, 7.0Hz, 1H), 4.07-4.17(m, 1H).
   IR(neat):
      3387, 2927, 2854, 1713, 1450, 1375, 1278, 1137, 1046, 969, 893, 681cm⁻¹.

### Example 24

### Preparations of 3-thia-9-deoxy-9β-bromo-16,17,18,19,20-pentanor-15-cyclohexyl-13,14-dihydro-PGF₁α and methyl ester thereof

(1) Following a manner similar to that of Example 13(1) using 3-thia-16,17,18,19,20-pentanor-15-cyclohexyl-13,14-dihydro-PGF₁α methyl ester 11,15-bis(t-butyldimethylsilyl ether) obtained in Example 23(3), 3-thia-9-deoxy-9β-bromo-16,17,18,19,20-pentanor-15-cyclohexyl-13,14-dihydro-PGF₁α methyl ester 11,15-bis(t-butyldimethylsilyl ether) was obtained.
   ¹H-NMR(CDCl₃, 200MHz) δ ppm; 0.02, 0.03, 0.04, 0.05 and 0.06(5s, 12H), 0.70-1.90(m, 41H), 2.04-2.20(m, 2H), 2.57-2.70(m, 2H), 3.23(s, 2H), 3.32-3.43(m, 1H), 3.74(s, 3H), 3.90-4.15(m, 2H).
   IR(neat):
      2929, 2855, 1741, 1472, 1463, 1361, 1256, 1090, 1072, 1007, 940, 900, 836, 774, 669cm⁻¹.
(2) Following a manner similar to that of Example 1(5) using the compound obtained in (1), 3-thia-9-deoxy-9β-bromo-16,17,18,19,20-pentanor-15-cyclohexyl-13,14-dihydro-PGF₁α methyl ester was obtained.
   ¹H-NMR(CDCl₃, 300MHz) δ ppm; 0.90-1.93(m, 23H), 2.17-2.30(m, 2H), 2.56-2.75(m, 2H), 3.23(br s, 2H), 3.34-3.45(m, 1H), 3.75(s, 3H), 4.00-4.16(m, 2H).
   IR(neat):
      3369, 2926, 2853, 1735, 1450, 1283, 1136, 1012, 969, 893, 702cm⁻¹.
(3) Following a manner similar to that of Example 1(6) using the compound obtained in (2), 3-thia-9-deoxy-9β-bromo-16,17,18,19,20-pentanor-15-cyclohexyl-13,14- dihydro-PGF₁α was obtained.
   ¹H-NMR(CDCl₃, 300MHz) δ ppm; 0.85-1.95(m, 23H), 2.17-2.34(m, 2H), 2.58-2.88(m, 2H), 3.05-3.35(br, 2H), 3.43-3.57(m, 1H), 3.70(br s, 3H), 4.02-4.17(m, 2H).
   IR(neat):
      3392, 2927, 2853, 1713, 1450, 1375, 1266, 1046, 969, 893, 680cm⁻¹.

### Example 25

### Preparations of 3-thia-9-deoxy-9β-chloro-16,17,18,19,20-pentanor-15-cyclohexyl-PGF₁α and methyl ester thereof

(1) To a solution of (3R)-1-iodo-3-(t-butyldimethylsiloxy)-3-cyclohexyl-1-propene (2.66 g) in 28 ml of ether was added t-butyl lithium (1.7 M, pentane solution, 8.24 ml) at -78°C, followed by stirring at the same temperature for an hour. After addition of lithium 2-thienylcyanocuprate (0.25 M, tetrahydrofuran solution, 39.2 ml), the mixture was stirred at the same temperature for 20 minutes, (4R)-2-(N,N-dimethylamino)methyl-4-(t-butyldimethylsiloxy)cyclopent-2-en-1-one (0.25 M, ether solution, 28 ml) was added thereto, and the temperature was elevated to 0°C with stirring over a period of 1.5 hours. To the reaction solution were added n-hexane (70 ml) and a saturated aqueous ammonium chloride solution (105 ml), the mixture was extracted with n-hexane, and the extract was washed with a saturated aqueous sodium chloride solution, dried, concentrated and purified by silica gel column chromatography (eluent; hexane : ethyl acetate = 30:1) to give 910 mg of (3R,4R)-2-methylene-3-[(3'R)-3'-(t-butyldimethylsiloxy)-3'-cyclohexyl-1'-propenyl]-4-(t-butyldimethylsiloxy)cyclopentan-1-one.
(2) Following a manner similar to that of Example 1(2) using the compound obtained in (1), 3-thia-16,17,18,19,20-pentanor-15-cyclohexyl-PGE₁ methyl ester 11,15-bis(t-butyldimethylsilyl ether) was obtained.
   ¹H-NMR(CDCl₃, 200MHz) δ ppm; -0.01(s, 3H), 0.04(s, 3H), 0.05(s, 3H), 0.06(s, 3H), 0.73-2.02(m, 18H), 0.88(s, 9H), 0.90(s, 9H), 2.17(dd, J=18.3, 7.9Hz, 1H), 2.40-2.81(m, 2H), 2.61(t, J=7.0Hz, 2H), 3.21(s, 2H), 3.74(s, 3H), 3.77-3.93(m, 1H), 4.11(dd, J=7.2, 2.8Hz, 1H), 5.39-5.65(m, 2H).
   IR(neat):
      2952, 2929, 2856, 1744, 1472, 1463, 1437, 1408, 1385, 1361, 1278, 1255, 1117, 1008, 974, 838, 777, 670cm⁻¹.
(3) Following a manner similar to that of Example 1(3) using the compound obtained in (1), 3-thia-16,17,18,19,20-pentanor-15-cyclohexyl-PGF₁α methyl ester 11,15-bis(t-butyldimethylsilyl ether) and 3-thia-16,17,18,19,20-pentanor-15-cyclohexyl-PGF₁β methyl ester 11,15-bis(t-butyldimethylsilyl ether) were obtained.
   ¹H-NMR(CDCl₃, 200MHz) δ ppm; -0.01(s, 3H), 0.03(s, 3H), 0.06(s, 6H), 0.76-1.97(m, 20H), 0.88(s, 9H), 0.89(s, 9H), 2.17-2.31(m, 1H), 2.63(t, J=7.1Hz, 2H), 3.22(s, 2H), 3.71-3.82(m, 1H), 3.73(s, 3H), 4.01-4.19(m, 2H), 5.22-5.49(m, 2H).
   IR(neat):
      3523, 2929, 2856, 1740, 1472, 1463, 1436, 1408, 1386, 1361, 1278, 1256, 1099, 1067, 1006, 974, 940, 924, 900, 837, 776, 668cm⁻¹.

   3-Thia-16,17,18,19,20-pentanor-15-cyclohexyl-PGF₁β methyl ester 11,15-bis(t-butyldimethylsilyl ether)
   ¹H-NMR(CDCl₃, 200MHz) δ ppm; -0.01 and 0.02(2s, 12H), 0.71-2.07(m, 21H), 0.86(s, 9H), 0.89(s, 9H), 2.67(t, J=7.0Hz, 2H), 3.22(s, 2H), 3.73(s, 3H), 3.74-3.83(m, 1H), 3.95-4.08(m, 2H), 5.40-5.47(m, 2H).
   IR(neat):
      3436, 2953, 2928, 2856, 1740, 1472, 1463, 1387, 1361, 1279, 1255, 1121, 1069, 1007, 974, 900, 837, 776, 671cm⁻¹.
(4) Following a manner similar to that of Example 1(4) using 3-thia-16,17,18,19,20-pentanor-15-cyclohexyl-PGF₁α methyl ester 11,15-bis(t-butyldimethylsilyl ether) obtained in (3), 3-thia-9-deoxy-9β-chloro-16,17,18,19,20-pentanor-15-cyclohexyl-PGF₁α methyl ester 11,15-bis(t-butyldimethylsilyl ether) was obtained.
   ¹H-NMR(CDCl₃, 200MHz) δ ppm; -0.01(s, 6H), 0.03(s, 6H), 0.72-1.86(m, 18H), 0.86(s, 9H), 0.89(s, 9H), 2.01-2.15(m, 2H), 2.10(ddd, J=7.4, 5.6, 1.2Hz, 1H), 2.62(t, J=7.1Hz, 2H), 3.21(s, 2H), 3.73(s, 3H), 3.74-3.83(m, 1H), 3.93-4.17(m, 2H), 5.27-5.57(m, 1H), 5.43(dd, J=4.9, 2.1Hz, 1H).
   IR(neat):
      2929, 2856, 1741, 1472, 1463, 1437, 1408, 1385, 1361, 1278, 1256, 1098, 1071, 1007, 974, 900, 837, 776, 672cm⁻¹.
(5) Following a manner similar to that of Example 1(5) using the compound obtained in (4), 3-thia-9-deoxy-9β-chloro-16,17,18,19,20-pentanor-15-cyclohexyl-PGF₁α methyl ester was obtained.
   ¹H-NMR(CDCl₃, 300MHz) δ ppm; 0.85-2.33(m, 20H), 2.27(ddd, J=14.2, 7.1, 5.3Hz, 1H), 2.62(t, J=7.1Hz, 2H), 3.22(s, 2H), 3.74(s, 3H), 3.78-3.88(m, 1H), 3.96-4.05(m, 1H), 4.07-4.20(m, 1H), 5.46-5.68(m, 2H).
   IR(KBr):
      3369, 2926, 2853, 1738, 1437, 1385, 1281, 1134, 1085, 1011, 973, 892, 756cm⁻¹.
(6) Following a manner similar to that of Example 1(6) using the compound obtained in (5), 3-thia-9-deoxy-9β-chloro-16,17,18,19,20-pentanor-15-cyclohexyl-PGF₁α was obtained.
   ¹H-NMR(CDCl₃, 300MHz) δ ppm; 0.82-2.10(m, 19H), 2.15-2.33(m, 1H), 2.27(ddd, J=14.1, 7.2, 5.4Hz, 1H), 2.56-2.73(m, 2H), 3.21(br s, 2H), 3.87-3.94(m, 1H), 3.96-4.06(m, 1H), 4.08-4.24(m, 1H), 5.43-5.77(m, 2H).
   IR(neat):
      3369, 2927, 2853, 1712, 1450, 1385, 1284, 1133, 1082, 974, 892, 757cm⁻¹.

### INDUSTRIAL APPLICABILITY

The compounds of the present invention have a potent lowering action of intraocular pressure with little side effects, and therefore they are useful for the treatment of glaucoma and other diseases and symptoms which are required to lower intraocular pressure.

## Claims

1. A prostaglandin derivative represented by Formula (I): 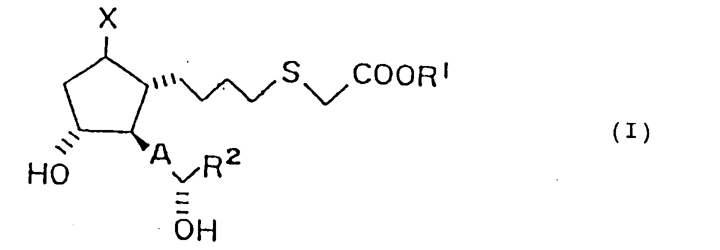 wherein R¹ is a hydrogen atom, an alkyl group having 1 to 10 carbon atoms or a cycloalkyl group having 3 to 10 carbon atoms, and R² is a cycloalkyl group having 3 to 10 carbon atoms, a cycloalkyl group having 3 to 10 carbon atoms substituted by a methyl group, an alkyl group having 1 to 3 carbon atoms substituted by a cycloalkyl group having 3 to 10 carbon atoms, a branched alkyl group having 5 to 10 carbon atoms or a branched alkenyl group having 5 to 10 carbon atoms, X is a halogen atom substituted at the α- or β-configuration, and A is an ethylene group, a vinylene group or an ethynylene group, or a salt thereof.

2. The prostaglandin derivative according to Claim 1 wherein R¹ is a hydrogen atom or an alkyl group having 1 to 6 carbon atoms, and A is an ethynylene group, or a salt thereof.

3. The prostaglandin derivative according to Claim 1 or 2 wherein R² is a cycloalkyl group having 3 to 8 carbon atoms, or an alkyl group having 1 to 3 carbon atoms substituted by a cycloalkyl group having 5 to 8 carbon atoms, or a salt thereof.

4. The prostaglandin derivative according to any one of Claim 1, 2 or 3 selected from the group consisting of 3-thia-9-deoxy-9β-chloro-16,17,18,19,20-pentanor-15-cyclohexyl-13,14-didehydro-PGF₁α; 3-thia-9-deoxy-9β-chloro-16,17,18,19,20-pentanor-15-cyclohexyl-13,14-didehydro-PGF₁α isopropyl ester; 3-thia-9-deoxy-9β-chloro-16,17,18,19,20-pentanor-15-cycloheptyl-13,14-didehydro-PGF₁α; 3-thia-9-deoxy-9β-bromo-16,17,18,19,20-pentanor-15-cyclohexyl-13,14-didehydro-PGF₁α methyl ester; 3-thia-9-deoxy-9β-bromo-16,17,18,19,20-pentanor-15-cyclohexyl-13,14-didehydro-PGF₁α and 3-thia-9-deoxy-9-fluoro-16,17,18,19,20-pentanor-15-cyclohexyl-13,14-didehydro-PGF₁, or the salt thereof.

5. The prostaglandin derivative or the salt thereof according to any one of Claims 1 to 4 for use as an effective ingredient of a pharmaceutical composition.

6. A pharmaceutical composition for lowering intraocular pressure comprising the prostaglandin derivative or the salt thereof according to any one of Claims 1 to 4 as an effective ingredient.

7. Use of a prostaglandin derivative or the salt thereof of any one of Claims 1 to 4 for the manufacture of a pharmaceutical composition for lowering intraocular pressure.

8. A method for lowering intraocular pressure in a human comprising administering an effective amount of the prostaglandin derivative or the salt thereof of any one of Claims 1 to 4 or a salt thereof as an effective ingredient to the human.
